(19) Europäisches Patentamt
European Patent Office
Office européen des brevets

(11) **EP 4 245 362 A1**

(12) **EUROPEAN PATENT APPLICATION**
published in accordance with Art. 153(4) EPC

(43) Date of publication:
**20.09.2023 Bulletin 2023/38**

(21) Application number: **21890827.5**

(22) Date of filing: **26.09.2021**

(51) International Patent Classification (IPC):
**A61N 5/067** *(2006.01)*        **A61B 18/20** *(2006.01)*

(52) Cooperative Patent Classification (CPC):
**A61N 5/067;** A61B 18/20

(86) International application number:
**PCT/CN2021/120558**

(87) International publication number:
**WO 2022/100299 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.11.2020   CN 202011251181**
**04.12.2020   CN 202011411381**
**21.12.2020   CN 202011519115**
**28.12.2020   CN 202011582205**

(71) Applicant: **Suzhou Microport Rehabtech (Group) Co., Ltd.**
**Suzhou, Jiangsu 215000 (CN)**

(72) Inventors:
• **XIE, Fei**
**Suzhou, Jiangsu 215000 (CN)**
• **HU, Jun**
**Suzhou, Jiangsu 215000 (CN)**
• **DI, Pei**
**Suzhou, Jiangsu 215000 (CN)**
• **LUO, Yi**
**Suzhou, Jiangsu 215000 (CN)**

(74) Representative: **Lavoix**
**Bayerstraße 83**
**80335 München (DE)**

(54) **LASER THERAPY DEVICE AND STORAGE MEDIUM**

(57)     The present invention provides a laser therapy device and a storage medium. The laser therapy device comprises a controller, a laser generator, and a driving power supply. The driving power supply is connected to the controller, and the laser generator is connected to the driving power supply. The controller is configured to receive an instruction and send out a control signal. The laser generator is configured to emit pulsed laser light, and the laser generator has at least two different laser generation modes. The driving power supply is configured to receive the control signals, and drive, according to the control signal, the laser generator to emit pulsed laser light in a corresponding generation mode.

Fig. 1

**Description**

**TECHNICAL FIELD**

The invention relates to the technical field of laser, in particular to a laser therapy device and a storage medium.

**BACKGROUND**

Compared with ordinary light, laser light does not disperse but goes straight forward, and has the characteristic of being able to achieve a strong output in a short time with a single wavelength. Laser light is a high-output non-ionized light with excellent monochromaticity and non-dispersion characteristics. Therefore, after the laser light is absorbed by the skin tissue, it will cause heating and photochemical reactions.

Laser therapy is a non-invasive treatment technique that helps reduce pain and inflammation, and can be safely used as an adjunct to or alternative of medication. This pain relief treatment has been approved by the Food and Drug Administration (FDA), allowing patients to have alternative medicine and surgery options. According to the different designs from different manufacturers, it can be divided into contact and non-contact treatments. The design of the contact treatment allows the therapist to apply a physical manipulation therapy at the same time as the laser treatment, so that the patient can receive laser treatment and physical manipulation therapy at the same time.

Effective laser therapy is a direct function of laser power and irradiation dose, giving the patient the optimal therapeutic dose to produce positive results. Laser therapy provides a greater depth of tissue penetration and ultimately delivers a dose to the target tissue that achieves a good therapeutic effect. Higher power also yields faster treatment times and provides therapeutic benefits that lower power lasers cannot. Therefore, the advantage of laser therapy device is that it is effective for difficult diseases, can be an alternative treatment plan for surgery, has faster treatment time, is also a simple non-invasive treatment method and is supported by scientific evidence.

The current laser therapy device uses a single laser generator to generate single-wavelength laser for treatment, and controls the on-off of the laser transmitter by relying on a conventional power driver to achieve the purpose of transmitting laser light. However, the laser light is output in a single mode and controlled in a single means, resulting in an impossibility in applying different laser lights to different patients to get a better treatment plan.

In an aspect, the existing laser therapy devices currently on the market do not have special treatment methods for different treatment parts, and all of them are treated by physical therapists or therapists according to the parts of human muscle pain. This kind of treatment method depends too much on human judgment and experience, and different muscle groups should have different treatment methods. Existing laser therapy devices usually only have one mode, and no matter which part or which muscle group receives the same treatment method, it cannot achieve better treatment goals. Moreover, the number and size of bones in different parts of the human body will also affect the absorption of laser energy. However, the existing equipment uses the same treatment method for physical therapy, so it cannot achieve better treatment goals.

In another aspect, the existing laser therapy devices currently on the market do not have treatment options for different therapeutic effects and different treatment tissues. The usual practice is for physiotherapists or therapists to treat according to the position of human muscle pain. This kind of treatment method depends too much on human judgment and experience, and there should be different treatment methods for different therapeutic effects and different treatment tissues. Existing laser therapy devices usually have only one mode, and a same treatment is applied to different parts such as muscles and bones, which cannot achieve better treatment goals. Moreover, laser therapy device may be used to achieve different therapeutic effects, such as pain relief and inflammation elimination, and different parts of the human body, such as muscles and bones, have different absorption of laser energy due to their different biological structures. However, the existing devices all use the same treatment modality for physical therapy, so the therapeutic effect is not good.

In another aspect, the existing laser therapy devices currently on the market do not have treatment options for different edematous tissues. It is common practice for a physical therapist or therapist to treat according to the edema part of the human body. This treatment method relies too much on human judgment and experience, and there should be different treatment methods for the edema of the four different tissues of tendon, muscle, joint and bone. The existing laser therapy device usually has only one mode, a same treatment is applied to tendon edema, muscle edema, joint edema, and bone edema, which cannot achieve better treatment goals. Moreover, when using laser therapy device to treat tendon edema, muscle edema, joint edema, and bone edema, the absorption of laser energy is also different due to the different biological structures of tendons, muscles, joints, and bones in the human body. However, the existing devices all use the same treatment modality for physical therapy, so the therapeutic effect is not good.

## SUMMARY OF THE INVENTION

An object of the present invention is to provide a laser therapy device and a storage medium, which can provide multiple laser generation modes, so that different laser therapeutic plans can be applied to different patients.

In order to achieve the above object, the present invention provides a laser therapy device, including a controller, a laser generator and a driving power supply; the driving power supply is connected to the controller, and the laser generator is connected to the driving power supply;

the controller is configured to receive an instruction and send out a control signal;
the laser generator is configured to emit pulsed laser light, and the laser generator has at least two different laser generation modes;
the driving power supply is configured to receive the control signal, and drive, according to the control signal, the laser generator to emit pulsed laser light in a corresponding laser generation mode.

In order to achieve the above object, the present invention also provides a storage medium in which a computer program is stored, and when the computer program is executed by a processor, it realizes:

receiving an instruction and sending out a control signal;
driving the laser generator, according to the control signal, to emit pulsed laser light in at least two different laser generation modes.

Compared with the prior art, the laser therapy device and storage medium provided by the present invention have the following advantages:

1. Since the laser generator in an embodiment of the present invention has at least two different laser generation modes, it is possible to use, according to specific conditions of the patient, any one of the at least two different laser generation modes for separate treatment or any combination of them for combined treatment, so that patients can be treated in a targeted manner, and the therapeutic effect and application range of the laser therapy device can be effectively improved.

2. The laser therapy device provided in other embodiments of the present invention includes a controller, a laser generator and a driving power supply. The laser generator has at least three different laser generation modes, and the correspondence between treatment parts and the laser generation modes of the laser generator is pre-stored in the controller. Therefore, it is possible to use any one of the at least three different laser generation modes for separate treatment or any combination of them for combined treatment according to the specific conditions of the patient's treatment parts, the therapeutic effect the patient wants to achieve, and/or the specific conditions of the treatment tissue, so that different treatment plans can be used for different treatment parts, which effectively improves the therapeutic effect and application range of the laser therapy device.

3. The laser therapy device provided in another embodiment of the present invention includes a controller, an input device, a laser generator and a driving power supply which are connected with each other. The laser generator has at least two different laser generation modes, and the correspondence between instructions related to edematous tissues and the laser generation modes of the laser generator is pre-stored in the controller. Therefore, it is possible to use any combination of the at least two different laser generation modes for combined treatment according to the specific conditions of the patient's edematous tissue, so that different edematous tissues can be treated with corresponding plans, which effectively improves the therapeutic effect and application range of the laser therapy device.

## BRIEF DESCRIPTION OF THE DRAWINGS

Fig. 1 is a structural block diagram of a laser therapy device according to an embodiment of the present invention;
Fig. 2 is a waveform diagram in the harmonic pulse mode according to an embodiment of the present invention;
Fig. 3 is a schematic diagram showing the interface display in the harmonic pulse mode according to an embodiment of the present invention;
Fig. 4 is a waveform diagram in a fixed pulse mode according to an embodiment of the present invention;
Fig. 5 is a schematic diagram showing the interface display in the fixed pulse mode according to an embodiment of the present invention;
Fig. 6 is a waveform diagram in the super pulse mode according to an embodiment of the present invention;
Fig. 7 is a schematic diagram showing the interface display in the super pulse mode according to an embodiment of the present invention;

Fig. 8 is a process flow chart of the laser therapy device according to an embodiment of the present invention;

Fig. 9 is a schematic diagram showing an interface display about patient age according to an embodiment of the present invention;

Fig. 10 is a schematic diagram showing the correspondence between the laser output energies for different modes and patient ages when the different modes are used in combination according to an embodiment of the present invention;

Fig. 11 is a schematic diagram showing an interface display about treatment parts according to an embodiment of the present invention;

Fig. 12 is a schematic diagram showing a comparison of laser treatment times for different treatment parts according to an embodiment of the present invention;

Fig. 13 is a schematic diagram showing a comparison of laser output powers for different treatment parts according an embodiment of the present invention;

Fig. 14 is a schematic diagram showing a comparison of laser output energies for different treatment parts according to an embodiment of the present invention;

Fig. 15 is a schematic diagram showing an interface display about optional therapeutic effects and treatment tissues according to an embodiment of the present invention;

Fig. 16 is a schematic diagram showing a comparison of laser treatment times for different therapeutic effects and different treatment tissues according to an embodiment of the present invention;

Fig. 17 is a schematic diagram showing a comparison of laser output powers for different therapeutic effects and different treatment tissues according to an embodiment of the present invention;

Fig. 18 is a schematic diagram showing a comparison of laser output energies for different therapeutic effects and different treatment tissues according to an embodiment of the present invention;

Fig. 19 is a schematic diagram showing an interface display about edematous tissues according to an embodiment of the present invention;

Fig. 20 is a schematic diagram showing a comparison of laser treatment times for different edematous tissues according to an embodiment of the present invention;

Fig. 21 is a schematic diagram showing a comparison of laser output powers for different edematous tissues according to an embodiment of the present invention;

Fig. 22 is a schematic diagram showing a comparison of laser output energies for different edematous tissues according to an embodiment of the present invention.

List of Reference Numerals:

100, controller; 200, laser generator; 300, driving power supply; 400, input device; 500, temperature sensor.

## DETAILED DESCRIPTION

The laser therapy device and storage medium according to the present invention will be described in greater detail below with reference to specific embodiments which are to be read in conjunction with the accompanying drawings. Advantages and features of the present invention will be apparent from the description. It is noted that the figures are provided in a very simplified form not necessarily presented to scale, with their only intention to facilitate convenience and clarity in explaining the disclosed embodiments. In order to make the objects, features and advantages of the present invention more comprehensible, please refer to the accompanying drawings. It should be noted that the structures, proportions, sizes, etc. shown in the drawings attached to this specification are only used to match the content disclosed in the specification, for those who are familiar with this technology to understand and read, and are not used to limit the implementation of the present invention, so it has no technical substantive meaning, and any modification of structure, change of proportional relationship or adjustment of size without affecting the effect and purpose of the present invention shall still fall within the scope of the present invention.

It should be noted that in this specification, relational terms such as first and second are only used to distinguish one entity or operation from another entity or operation, and do not necessarily require or imply that there is such actual relationship or order between these entities or operations. Furthermore, the term "comprise", "include" or any other variation thereof is intended to cover a non-exclusive inclusion such that a process, method, object, or device comprising a set of elements includes not only those elements, but also other elements not expressly listed, or also includes elements inherent in such a process, method, object, or device. Without further limitations, an element defined by the phrase "comprising a ..." does not exclude the presence of additional identical elements in the process, method, object or device comprising said element.

In describing the present invention, it is to be understood that the terms "center", "longitudinal", "transverse", "length", "width", "thickness", "upper", "lower", "front", " back", "left", "right", "vertical", "horizontal", "top", "bottom", "inner", "outer", "axial", "radial", "circumferential" refers to the orientation or positional relationship shown in the drawings, which is only for the convenience of describing the present invention and simplifying the description, rather than indicating or implying that the referred device or element must have the specific orientation, be constructed or operated in the specific orientation, and thus it should not be construed as limiting the invention. In the description of the present invention, unless otherwise specified, "plurality" means two or more.

In the description of the present invention, unless otherwise clearly specified and limited, the terms "installation", "connection", "couple" and "fixation" should be understood in a broad sense, for example, it can be a fixed connection or a detachable connection , or integrated; it can be mechanically connected or electrically connected; it can be directly connected or indirectly connected through an intermediary, and it can be the internal communication of two components or the interaction relationship between two components. Those of ordinary skill in the art can understand the specific meanings of the above terms in the present invention in specific situations.

In the present invention, unless otherwise clearly specified and limited, a first feature being "on" or "under" a second feature may be interpreted as the first feature directly contacting the second feature, and may also be interpreted as the first feature contacting the second feature through another feature between them instead of directly contacting. Moreover, the first feature being "on", "above" or "over" the second feature may be interpreted as the first feature being directly above or obliquely above the second feature, or simply interpreted as the first feature being horizontally higher than the second feature. The first feature is "below", "beneath" or "under" the second feature may be interpreted as the first feature being directly below or obliquely below the second feature, or simply interpreted as the first feature being horizontally lower than the second feature.

EMBODIMENT 1

The core idea of this embodiment is to provide a laser therapy device and a storage medium, which can provide multiple laser generation modes, so that different laser treatment plans can be applied to different patients.

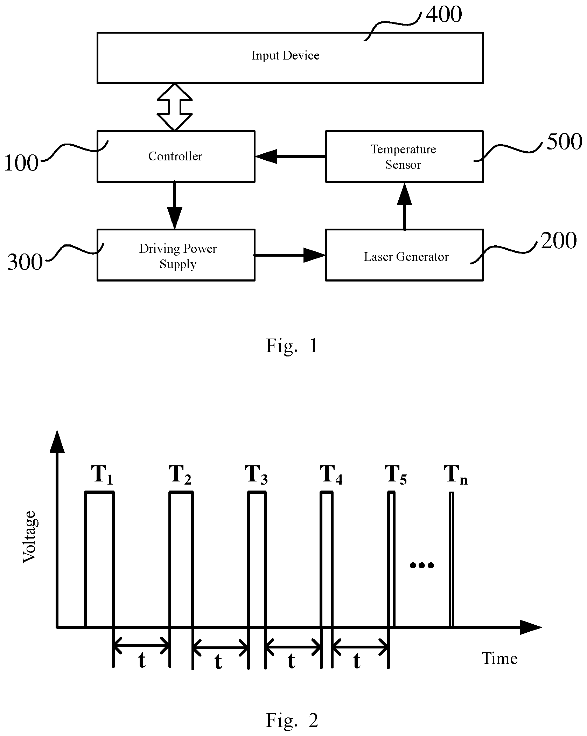

In order to achieve the above idea, the present embodiment provides a laser therapy device. Please refer to Fig. 1, which schematically shows a structural block diagram of a laser therapy device according to an embodiment of the present invention. As shown in Fig. 1, the laser therapy device comprises a controller 100, a laser generator 200, a driving power supply 300 and an input device 400. The driving power supply 300 and the input device 400 are connected to the controller 100, and the laser generator 200 is connected to the driving power supply 300.

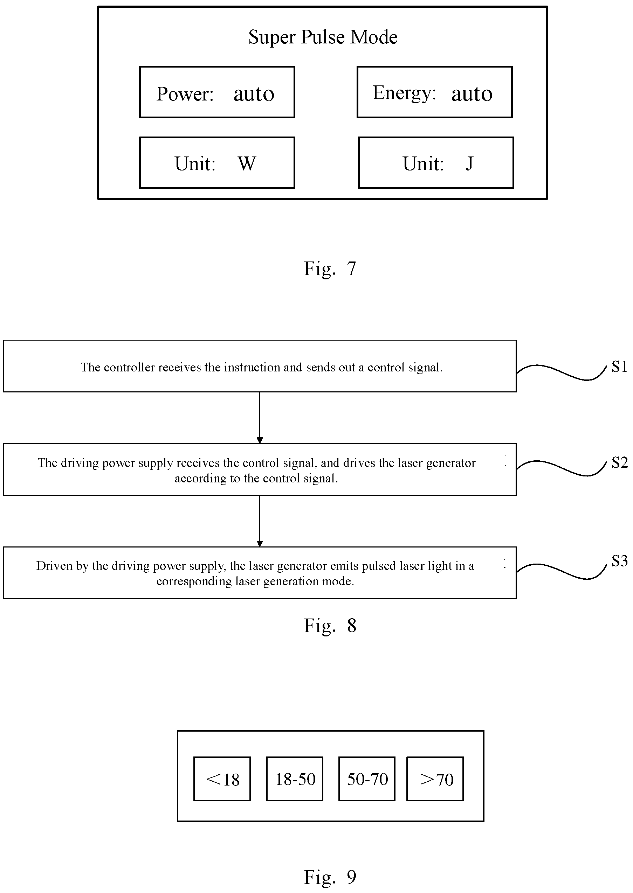

The laser generator 200 is configured to emit pulsed laser light, and has at least two different laser generation modes. The controller 100 is configured to receive an instruction and send out a control signal. The driving power supply 300 is configured to receive the control signal, and drive, according to the control signal, the laser generator 200 to emit pulsed laser light in a corresponding laser generation mode. Since the laser generator 200 has at least two different laser generation modes, any one of the at least two different laser generation modes can be used for individual treatment or any combination of them can be used for combined treatment, according to the specific conditions of a patient, so that the patient can be treated in a targeted manner, and the therapeutic effect and application range of the laser therapy device can be effectively improved. Specifically, the user can control the laser generating mode of the laser generator 200 according to the specific conditions of the patient, and send a corresponding instruction to the controller 100 to execute the instruction. Please refer to Fig. 8, which schematically shows the process flow chart of the laser therapy device according to an embodiment of the present invention. As shown in Fig. 8, when the user provides an instruction according to the specific conditions of the patient, the laser therapy device operates according to the following steps.

Step S1, the controller receives the instruction and sends out a control signal;
Step S2, the driving power supply receives the control signal, and drives the laser generator according to the control signal;
Step S3, driven by the driving power supply, the laser generator emits pulsed laser light in a corresponding laser generation mode.

Preferably, the driving power supply 300 is an adjustable constant current source. After receiving the control signal, the driving power supply 300 converts the control signal into a current signal to drive the laser generator 200 to emit a pulsed laser light in a corresponding mode.

Preferably, the laser therapy device includes a display screen connected to the controller 100 which is configured, as an output device and/or an input device 400, for interface display and instruction delivery. In this way, the user can input corresponding instructions through the display screen, and the display screen sends the instructions to the controller 100, so that the operation is more convenient.

Preferably, the display screen is an LCD touch screen. Therefore, the use of a touch screen can facilitate human-computer interaction. It should be noted that, in some other implementations, the display screen may be a button-type or hand-written display screen, which is not limited in the present invention.

Preferably, the laser therapy device includes a temperature sensor 500 connected to the controller 100. The temperature sensor 500 is configured to detect the temperature of the laser generator 200 and transmit the detected temperature result to the controller 100. Thus, the temperature of the laser generator 200 can be detected in real time by the temperature sensor 500, and the controller 100 can determine whether the laser generator 200 is in an overheated state according to the temperature result fed back from the temperature sensor 500. In this way, the laser generator 200 can be protected from overheating so that the laser generator 200 is prevented from being damaged due to overheating. Moreover, the controller 100 can adjust the temperature of the treatment parts of human body based on the temperature information to prevent the human body form an excessive temperature.

Preferably, the laser generator 200 includes three laser generation modes that are harmonic pulse mode, fixed pulse mode and super pulse mode. Thus, according to the specific conditions of the patient, the user, such as a doctor, can choose any one of the three modes to treat the patient, or use any two of the three modes to treat the patient separately in different time periods, or use these three modes to treat the patient separately in different time periods, in order to achieve the best therapeutic effect.

Preferably, please refer to Fig. 2, which schematically shows a waveform diagram in the harmonic pulse mode according to an embodiment of the present invention. As shown in Fig. 2, in the harmonic pulse mode, the laser generator 200 emits n pulses at an equal pulse interval in one period T, and the pulse widths of the n pulses are different, wherein n is a positive integer, and $n \geq 2$.

It is defined that the pulse width of the $N^{th}$ pulse emitted from the laser generator 200 in one period T is $T_N$, N=1, 2, 3, ... n, the pulse interval between the $(N+1)^{th}$ pulse and the $N^{th}$ pulse is t, the total time for the laser generator 200 to emit pulses is $T_{on}$, and the total intermittent time of the laser generator 200 is $T_{off}$, then there are:

$$T_{on} = \sum_{N=1}^{n} T_N \qquad (1)$$

$$T_{off} = (n-1) * t \qquad (2)$$

$$T = T_{on} + T_{off} \qquad (3)$$

Preferably, the pulse width $T_{N+1}$ of the $(N+1)^{th}$ pulse and the pulse width $T_N$ of the $N^{th}$ pulse satisfy the following equation:

$$T_{N+1} = \frac{1}{\theta} T_N \qquad (4)$$

wherein, $\theta$ is the average energy coefficient which is a positive integer equal to or greater than 2, and N is a positive integer.

Thus, according to the initial pulse width $T_1$ (i.e., the pulse width of the first pulse emitted from the laser generator 200 in one period) and the average energy coefficient, the pulse widths of other pulses can be determined. The initial pulse width $T_1$, pulse interval t and average energy coefficient $\theta$ and other parameters are preset in the laser therapy device, for example, before the laser therapy device leaves the factory. Thus, based on the various parameters preset in the laser generator 200, the laser generator 200 can output pulsed laser light in the harmonic pulse mode.

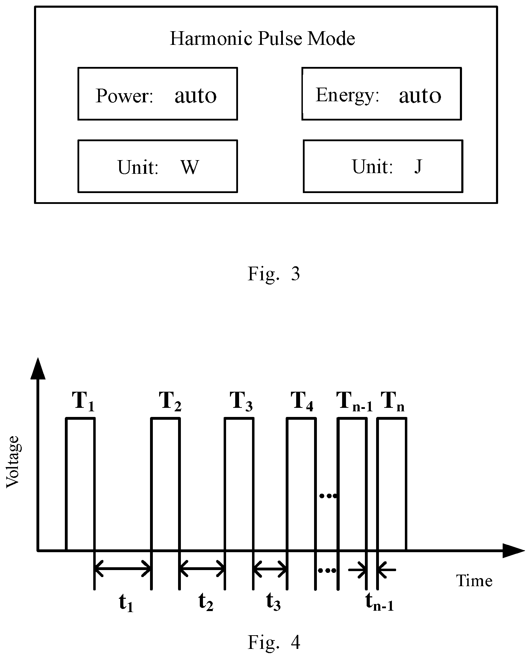

Please refer to Fig. 3, which schematically provides a schematic diagram of the interface display of the display screen in the harmonic pulse mode according to an embodiment of the present invention. As shown in Fig. 3, in the harmonic pulse mode, the total output energy and total output power of the laser light are automatically adjusted by the controller 100 according to preset parameters, which dispenses with manual adjustment by the user. In some embodiments, the content displayed on the interface in the harmonic pulse mode may not be provided to the user, but only for staff or product designers to debug.

Preferably, please refer to Fig. 4, which schematically shows a waveform diagram in a fixed pulse mode according to an embodiment of the present invention. As shown in Fig. 4, in a fixed pulse mode, the laser generator 200 emits n pulses with same pulse widths at different pulse intervals in one period, where n is a positive integer, and $n \geq 3$.

It is defined that the pulse width of the $N^{th}$ pulse emitted from the laser generator 200 in one period T is $T_N$, N=1, 2, 3, ... n, the pulse interval between the $(N+1)^{th}$ pulse and the $N^{th}$ pulse is $t_K$, K=1, 2, 3, ... n-1, the total time for the laser generator 200 to emit pulses is $T_{on}$, and the total intermittent time of the laser generator 200 is $T_{off}$, then there are:

$$T_1 = T_2 = T_3 = \ldots = T_n \tag{5}$$

$$T_{on} = n * T_1 \tag{6}$$

$$T_{off} = \sum_{K=1}^{n-1} t_K \tag{7}$$

$$T = T_{on} + T_{off} \tag{3}$$

Preferably, the pulse interval $t_k$ between the $(N+1)^{th}$ pulse and the $N^{th}$ pulse and the pulse interval $t_{k+1}$ between the $(N+2)^{th}$ pulse and the $(N+1)^{th}$ pulse satisfy the following equation:

$$t_{K+1} = \frac{1}{\xi} t_K \tag{8}$$

wherein, $\xi$ is the average power coefficient which is a positive integer equal to or greater than 2, N is a positive integer, and K is a positive integer.

Thus, according to the initial pulse interval ti (i.e., the pulse interval between the first pulse and the second pulse emitted from the pulsed laser generator 200 in one period) and the average power coefficient $\xi$, the pulse intervals of subsequent pulses can be determined. According to the received instruction, the controller 100 adjusts at least one of the parameters such as the initial pulse width $T_1$, number n of the pulses, average power coefficient $\xi$ and the initial pulse interval ti. Therefore, it is possible to output pulsed laser lights with different energies, powers and frequencies in the fixed pulse mode.

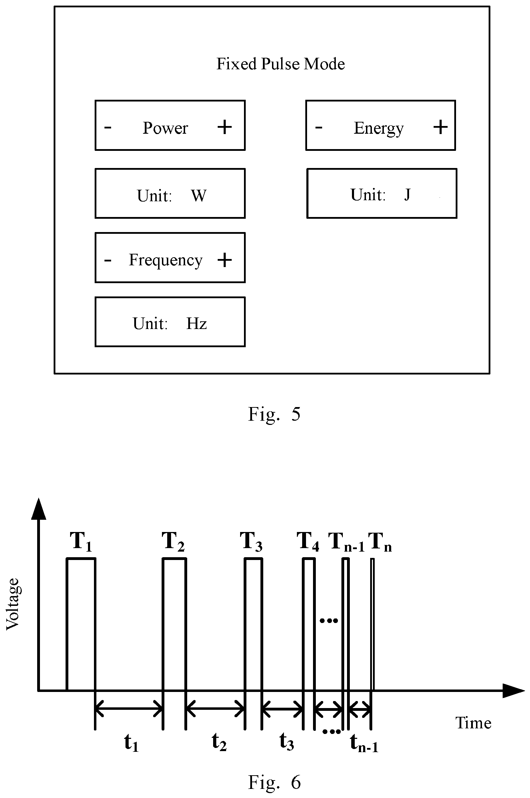

Please refer to Fig. 5, which schematically shows a schematic diagram of the interface display of the display screen in the fixed pulse mode according to an embodiment of the present invention. As shown in Fig. 5, in the fixed pulse mode, the total output energy, total output power and frequency of the laser light can be adjusted by the user according to actual needs. In some embodiments, the content displayed on the interface in the fixed pulse mode may not be provided to the user, but only for staff or product designers to debug.

Preferably, please refer to Fig. 6, which schematically shows a waveform diagram in the super pulse mode according to an embodiment of the present invention. As shown in Fig. 6, in the super pulse mode, the laser generator 200 emits n pulses with different pulse widths at different pulse intervals in one period, wherein n is a positive integer, and n A 3.

It is defined that the pulse width of the $N^{th}$ pulse emitted from the laser generator 200 in one period T is $T_N$, N=1, 2, 3, ... n, the pulse interval between the $(N+1)^{th}$ pulse and the $N^{th}$ pulse is $t_K$, K=1, 2, 3, ... n-1, the total time for the laser generator 200 to emit pulses is $T_{on}$, and the total intermittent time of the laser generator 200 is $T_{off}$, then there are:

$$T_{on} = \sum_{N=1}^{n} T_N \tag{1}$$

$$T_{off} = \sum_{K=1}^{n-1} t_K \tag{7}$$

$$T = T_{on} + T_{off} \tag{3}$$

Preferably, the pulse width $T_{N+1}$ of the $(N+1)^{th}$ pulse and the pulse width $T_N$ of the $N^{th}$ pulse satisfy the following equation:

$$T_{N+1} = \frac{1}{\theta} T_N \qquad\qquad (4)$$

wherein, $\theta$ is the average energy coefficient which is a positive integer equal to or greater than 2, and N is a positive integer.

Thus, according to the initial pulse width $T_1$ (i.e., the pulse width of the first pulse emitted from the laser generator 200 in one period) and the average energy coefficient $\theta$, the pulse widths of other pulses can be determined.

Preferably, the pulse interval $t_K$ between the $(N+1)^{th}$ pulse and the $N^{th}$ pulse and the pulse interval $T_{K+1}$ between the $(N+2)^{th}$ pulse and the $(N+1)^{th}$ pulse satisfy the following equation:

$$t_{K+1} = \frac{1}{\xi} t_K \qquad\qquad (8)$$

wherein, $\xi$ is the average power coefficient which is a positive integer equal to or greater than 2, N is a positive integer, and K is a positive integer.

Thus, according to the initial pulse interval ti (i.e., the pulse interval between the first pulse and the second pulse emitted from the pulsed laser generator 200 in one period) and the average power coefficient $\xi$, the pulse intervals of subsequent pulses can be determined.

In conclusion, in super pulse mode, by setting one or more of the parameters such as the initial pulse width $T_1$, number n of the pulses, average energy coefficient $\theta$, average power coefficient $\xi$ and the initial pulse interval ti, it is possible to output pulsed laser light with corresponding energy and power. In an embodiment of the present invention, the parameters are preset.

Please refer to Fig. 7, which schematically shows a schematic diagram of the interface display of the display screen in the super pulse mode according to an embodiment of the present invention. As shown in Fig. 7, in the super pulse mode, the total output energy and total output power of the laser light are automatically adjusted by the controller 100 according to preset parameters, which dispenses with manual adjustment by the user. In some embodiments, the content displayed on the interface in the super pulse mode may not be provided to the user, but only for staff or product designers to debug.

The operating principle and application scenarios of the laser therapy device according to this embodiment will be described below through two specific examples.

Example 1

At present, existing laser therapy devices do not differentiate people of different skin tones. With different skin tones, the skin absorbs laser energy differently. The deeper the skin tone, the higher the laser energy absorption rate.

Therefore, the laser therapy device according to this embodiment can provide different treatment plans for different skin tones. Depending on the patient skin tone, five different skin tones are defined as very fair, fair, yellow, brown, and dark brown. It should be noted that, in some other implementations, different skin tones may be classified in other ways.

In the laser therapy device according to this embodiment, the controller 100 is configured to control the laser generator according to the pre-stored correspondence between patient skin tones and the laser generation modes of the laser generator (that is, treatment plans for different skin tones).

Specifically, the controller 100 is configured to control the total irradiation time, total output power and total output energy of the laser light emitted from the laser generator to decrease as the patient skin tone becomes darker.

As the patient skin tone gets darker, the controller 100 is configured to control the respective laser irradiation times for the harmonic pulse mode, the fixed pulse mode and the super pulse mode to decrease, wherein the laser irradiation time for the fixed pulse mode decreases the most.

As the patient skin tone gets darker, the controller 100 is configured to control the respective laser output energies for the harmonic pulse mode, the fixed pulse mode and the super pulse mode to decrease, wherein the laser output energy for the fixed pulse mode decreases the most, and the laser output energy for the harmonic pulse mode decreases the second most, and the laser output energy for the super pulse mode decreases the least.

Thus, the user can select a corresponding treatment plan according to the patient skin tone, and send a corresponding instruction to the controller 100 through the display screen. For ease of operation, schematic diagrams of different skin tones are displayed on the display screen. Specifically, illustrations of typical characteristic colors for five different skin tones of very fair, fair, yellow, brown, and dark brown are displayed on the display screen, and the user can compare the actual skin tone of the patient according to the color icon prompted, and select the same or similar color icon to choose different skin tones.

Treatment options for different skin tones are as follows:

(1) As the patient skin tone gets darker, for example, from very fair to dark brown, the total treatment time of laser irradiation (that is, the total irradiation time) decreases, the total output power of the laser light decreases, and the total output energy of the laser light decreases. For people with very fair skin, the total treatment time is the longest, and the total output power and total output energy of the laser light are the highest. For people with dark brown skin, the total treatment time is the shortest, and the total output power and total output energy of the laser light are the lowest.

(2) As the patient skin tone gets darker, for example, from very fair to dark brown, the individual treatment time (i.e., irradiation time) for the three different laser generation modes (that are the harmonic pulse mode, fixed pulse mode and super pulse mode) decreases, the treatment time for the fixed pulse mode decreases the most.

(3) As the patient skin tone gets darker, for example, from very fair to dark brown, the output energy for the three different laser generation modes (that are harmonic pulse mode, fixed pulse mode and super pulse mode) decreases. The output energy for the fixed pulse mode decreases the most, the output energy for the harmonic pulse mode decreases the second most, and the output energy for the super pulse mode decreases the least.

(4) For different skin tones, three different laser generation modes (that are harmonic pulse mode, fixed pulse mode and super pulse mode) can be used alone or in combination.

Please refer to Tables 1 to 5 shown below, wherein Table 1 schematically shows the combined treatment plan of three different laser generation modes for very fair skin according to an embodiment of the present invention; Table 2 schematically shows the combined treatment plan of three different laser generation modes for fair skin according to an embodiment of the present invention; Table 3 schematically shows the combined treatment plan of three different laser generation modes for yellow skin according to an embodiment of the present invention; Table 4 schematically shows the combined treatment plan of three different laser generation modes for brown skin according to an embodiment of the present invention; Table 5 schematically shows the combined treatment plan of three different laser generation modes for dark brown skin according to an embodiment of the present invention.

(1) Skin Tone: very fair

Table 1: Treatment plan for very fair skin

| Stage | Time/s | Power/W | Energy/J | Model |
|-------|--------|---------|----------|-------|
| 1 | 28 | 5 | 56 | Fixed Pulse Mode |
| 2 | 150 | 5 | 105 | Harmonic Pulse Mode |
| 3 | 120 | 6 | 135 | Super Pulse Mode |
| Total | 298 | 16 | 296 | |

(2) Skin Tone: fairer

Table 2: Treatment plan for fair skin

| Stage | Time/s | Power/W | Energy/J | Model |
|-------|--------|---------|----------|-------|
| 1 | 25 | 4 | 42 | Fixed Pulse Mode |
| 2 | 149 | 4 | 103 | Harmonic Pulse Mode |
| 3 | 119 | 6 | 134 | Super Pulse Mode |
| Total | 293 | 14 | 279 | |

(3) Skin Tone: yellow

Table 3: Treatment plan for yellow skin

| Stage | Time/s | Power/W | Energy/J | Model |
|---|---|---|---|---|
| 1 | 23 | 3 | 40 | Fixed Pulse Mode |
| 2 | 148 | 4 | 100 | Harmonic Pulse Mode |
| 3 | 118 | 5 | 132 | Super Pulse Mode |
| Total | 289 | 12 | 272 | |

(4) Skin Tone: brown

Table 4: Treatment plan for brown skin

| Stage | Time/s | Power/W | Energy/J | Model |
|---|---|---|---|---|
| 1 | 21 | 2 | 21 | Fixed Pulse Mode |
| 2 | 146 | 3 | 90 | Harmonic Pulse Mode |
| 3 | 117 | 5 | 130 | Super Pulse Mode |
| Total | 284 | 10 | 241 | |

(5) Skin Tone: dark brown

Table 5: Treatment plan for dark brown skin

| Stage | Time/s | Power/W | Energy/J | Model |
|---|---|---|---|---|
| 1 | 14 | 1 | 20 | Fixed Pulse Mode |
| 2 | 144 | 2 | 85 | Harmonic Pulse Mode |
| 3 | 116 | 5 | 125 | Super Pulse Mode |
| Total | 274 | 8 | 230 | |

From Tables 1 to 5, it can be seen that, as the patient skin tone gets darker, the laser output energy and irradiation time for the fixed pulse mode, the harmonic pulse mode and the super pulse mode decrease; the output energy for the fixed pulse mode decreases the most, and the output energy for the harmonic pulse mode decreases the second most, and the output energy for the super pulse mode decreases the least; the laser irradiation time for the fixed pulse mode decreases the most.

Example 2

At present, the existing laser therapy device does not differentiate between people of different ages. As people get older, skin aging is inevitable, the original function of the skin declines, the number of the fibroblasts in the skin decreases, the metabolism of epidermal cells slows down, and the vitality of the cells themselves decreases. The process of skin aging will also accelerate with age. The quality and quantity of collagen and elastin in the dermis will decrease, the skin will lose its elasticity, and blood circulation will become poor. It can be seen that with different ages, the human body has different requirements for the amount of energy and energy application mode in the laser treatment process.

The laser therapy device according to this embodiment can provide different treatment plans for different ages. According to the patient age, four different age groups were defined as less than 18 years, 18 to 50 years, 50 to 70 years, and greater than 70 years. It should be noted that in some other implementations, different age groups may also be defined in other ways as required.

In the laser therapy device according to this embodiment, the controller 100 is configured to control the laser generator according to the pre-stored correspondence between patient age groups and the laser generation modes of the laser generator (that is, the treatment plans for different ages).

Specifically, as the patient age increases, the controller 100 is configured to control the total irradiation time, total output power and total output energy of the laser light emitted from the laser generator to increase.

As the patient age increases, the controller 100 is configured to control the respective laser irradiation times for the harmonic pulse mode, the fixed pulse mode and the super pulse mode to increase, and the laser irradiation time for the fixed pulse mode increases the most.

As the patient age increases, the controller 100 is configured to control the respective laser output energies for the harmonic pulse mode, the fixed pulse mode and the super pulse mode to increase, wherein the laser output energy for the fixed pulse mode increases the most, and the laser output energy for the harmonic pulse mode increases the second most, and the laser output energy for super pulse mode increases the least.

Thus, the user can select a corresponding treatment plan according to the age group of the patient, and send a corresponding instruction to the controller 100 through the display screen. In order to facilitate the operation, there are indicating figures for different age groups on the display screen to remind the user. The user can choose them according to the patient age as needed. The specific display interface is shown in Fig. 9.

Treatment options for different ages are as follows:

(1) As the patient age increases, for example, the age changes from less than 18 years old to more than 70 years old, the total treatment time of laser irradiation increases, the total output power of the laser light increases, and the total output energy of the laser light increases. For patients with an age less than 18 years old, the total treatment time is the shortest, and the total laser output power and total output energy of the laser light are the lowest. For patients with an age more than 70 years old, the total treatment time is longest, and the total output power and total output energy of the laser light are the highest.

(2) As the patient age increases, for example, the age changes from less than 18 years old to more than 70 years old, the individual treatment time for the three different treatment modes (that are the harmonic pulse mode, fixed pulse mode and super pulse mode) increases, and the treatment time for the fixed pulse mode increases the most.

(3) As the patient age increases, for example, the age changes from less than 18 years old to more than 70 years old, the output energy for the three different treatment modes (that are the harmonic pulse mode, fixed pulse mode and super pulse mode) increases. The output energy for the fixed pulse mode increases the most, the output energy for the harmonic pulse mode increases the second most, and the output energy for the super pulse mode increases the least.

(4) For different ages, the three different laser generation modes (that are the harmonic pulse mode, fixed pulse mode and super pulse mode) can be used alone or in combination.

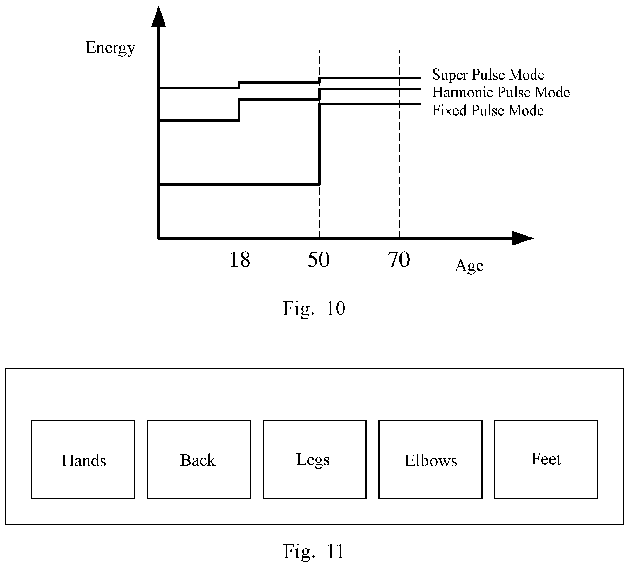

Please refer to Fig. 10, which schematically shows the correspondence between the laser output energies for different modes and the patient ages when the different modes are used in combination according to an embodiment of the present invention. As shown in Fig. 10, as the patient age increases, the laser output energies for the fixed pulse mode, the harmonic pulse mode and the super pulse mode all increase. The output energy for the fixed pulse mode increases the most, the output energy for the harmonic pulse mode increases the second most, and the output energy for the super pulse mode increases the least.

In order to achieve the above idea, this embodiment also provides a storage medium, in which a computer program is stored, and when the computer program is executed by a processor, it realizes:

receiving an instruction and sending out a control signal;
driving the laser generator, according to the control signal, to emit pulsed laser light in at least two different laser generation modes.

Since the laser generator in this embodiment has at least two different laser generation modes, it is possible to use, according to specific conditions of the patient, any one of the at least two different laser generation modes for separate treatment or any combination of them for combined treatment, so that patients can be treated in a targeted manner, and the therapeutic effect and application range of the laser therapy device can be effectively improved.

The storage medium in the embodiments of the present invention may be implemented as one of or any combination of a plurality of computer-readable mediums. The readable medium may be a computer readable signal medium or a computer readable storage medium. A computer-readable storage medium may be, for example, but not limited to, an electrical, magnetic, optical, electromagnetic, infrared, or semiconductor system, apparatus, or device, or any combination thereof. More specific examples (non-exhaustive list) of computer readable storage mediums include: electrical connection with one or more conductors, portable computer hard disk, hard disk, random access memory (RAM), read only memory (ROM), erasable programmable read-only memory (EPROM or flash memory), optical fiber, portable compact disk read-only memory (CD-ROM), optical storage device, magnetic storage device, or any suitable combination of the above. Herein, a computer-readable storage medium may be any tangible medium that contains or stores a program that can be used by or in combination with an instruction execution system, apparatus, or device.

A computer readable signal medium may include a data signal carrying computer readable program code in baseband or propagated as part of a carrier wave. Such propagated data signals may be in various forms, including but not limited to electromagnetic signals, optical signals, or any suitable combination of the foregoing. A computer-readable signal medium may be any computer-readable medium other than a computer-readable storage medium, which can send, propagate, or transmit a program for use by or in conjunction with an instruction execution system, apparatus, or device..

Computer program code for carrying out the operations of the present invention may be written in one or more programming languages, or combinations thereof, including object-oriented programming languages, such as Java, Smalltalk, C++, also including conventional procedural programming language, such as "C" or a similar programming language. The program code may execute entirely on the user's computer, partly on the user's computer, as a stand-alone software package, partly on the user's computer and partly on a remote computer, or entirely on the remote computer or server. In cases involving a remote computer, the remote computer may be connected to the user computer through any kind of network, including a local area network (LAN) or a wide area network (WAN), or may be connected to an external computer (for example through an Internet connection provided by an Internet service provider).

Preferably, the laser generator includes three laser generation modes that are the harmonic pulse mode, fixed pulse mode and super pulse mode.

Preferably, in the harmonic pulse mode, the laser generator emits n pulses at an equal pulse interval in one period, and the pulse widths of the n pulses are different, where n is a positive integer, and nN2.

Preferably, the pulse width $T_{N+1}$ of the $(N+1)^{th}$ pulse and the pulse width of the $N^{th}$ pulse $T_N$ satisfy the following equation:

$$T_{N+1} = \frac{1}{\theta} T_N \qquad (4)$$

wherein, $\theta$ is the average energy coefficient which is a positive integer equal to or greater than 2, and N is a positive integer.

Preferably, in the fixed pulse mode, the laser generator emits n pulses with same pulse widths at different pulse intervals in one period, where n is a positive integer, and n≥3.

Preferably, the pulse interval $t_k$ between the $(N+1)^{th}$ pulse and the $N^{th}$ pulse and the pulse interval $t_{k+1}$ between the $(N+2)^{th}$ pulse and the $(N+1)^{th}$ pulse satisfy the following equation:

$$t_{K+1} = \frac{1}{\xi} t_K \qquad (8)$$

wherein, $\xi$ is the average power coefficient which is a positive integer equal to or greater than 2, and N is a positive integer.

Preferably, in the super pulse mode, the laser generator emits n pulses with different pulse widths at different pulse intervals in one period, wherein n is a positive integer, and n≥3.

Preferably, the pulse width $T_{N+1}$ of the $(N+1)^{th}$ pulse and the pulse width $T_N$ of the $N^{th}$ pulse satisfy the following equation:

$$T_{N+1} = \frac{1}{\theta} T_N \qquad (4)$$

wherein, $\theta$ is the average energy coefficient which is a positive integer equal to or greater than 2, and N is a positive integer.

Preferably, the pulse interval $t_K$ between the $(N+1)^{th}$ pulse and the $N^{th}$ pulse and the pulse interval $T_{k+1}$ between the $(N+2)^{th}$ pulse and the $(N+1)^{th}$ pulse satisfy the following equation:

$$t_{K+1} = \frac{1}{\xi} t_K \qquad (8)$$

wherein, $\xi$ is the average power coefficient which is a positive integer equal to or greater than 2, and N is a positive integer.

Preferably, the correspondence between patient skin tones and the laser generation modes of the laser generator is pre-stored in the storage medium.

Preferably, as the patient skin tone gets darker, the total irradiation time, total output power and total output energy of the laser light emitted from the laser generator decrease.

Preferably, as the patient skin tone gets darker, the respective laser irradiation times for the harmonic pulse mode, the fixed pulse mode and the super pulse mode decrease, wherein the laser irradiation time for the fixed pulse mode decreases the most.

Preferably, as the patient skin tone gets darker, the respective laser output energies for the harmonic pulse mode, the fixed pulse mode and the super pulse mode decrease, wherein the laser output energy for the fixed pulse mode decreases the most, and the laser output energy for the harmonic pulse mode decreases the second most, the laser output energy for the super pulse mode decreases the least.

Preferably, the correspondence between patient age groups and the laser generation modes of the laser generator is pre-stored in the storage medium.

Preferably, as the patient age increases, the total irradiation time, total output power and total output energy of the laser light emitted from the laser generator increases.

Preferably, as the patient age increases, the respective laser irradiation times for the harmonic pulse mode, the fixed pulse mode and the super pulse mode increase, and the laser irradiation time for the fixed pulse mode increases the most.

Preferably, as the patient age increases, the respective laser output energies for the harmonic pulse mode, the fixed pulse mode and the super pulse mode increase, wherein the laser output energy for the fixed pulse mode increases the most, the laser output energy for the harmonic pulse mode increases the second most, and the laser output energy for the super pulse mode increases the least.

Preferably, when the computer program is executed by a processor, it further realizes:

obtaining the temperature of the laser generator, and determining whether the temperature is higher than a preset threshold, and if so, performing overheating protection on the laser generator.

In conclusion, compared with the prior art, the laser therapy device and storage medium according to this embodiment have the following advantages. Since the laser generator in this embodiment has at least two different laser generation modes, it is possible to use, according to specific conditions of the patient, any one of the at least two different laser generation modes for separate treatment or any combination of them for combined treatment, so that patients can be treated in a targeted manner, and the therapeutic effect and application range of the laser therapy device can be effectively improved.

EMBODIMENT 2

The main object for this embodiment is to provide a laser therapy device and a storage medium, which can provide multiple laser generation modes, so that different laser treatment plans can be applied to different treatment parts.

As to the similarities between Embodiment 2 and Embodiment 1, please refer to the description in Embodiment 1, and details are not repeated here. That is to say, content not described in Embodiment 2 may refer to Embodiment 1 or other embodiments of the present invention. The differences between Embodiment 2 and Embodiment 1 are mainly described below.

In order to achieve the above ideas, this embodiment provides a laser therapy device. Please refer to Fig. 1, which schematically shows a structural block diagram of a laser therapy device according to an embodiment of the present invention. For details, please refer to the description in Embodiment 1, which will not be repeated here.

The laser generator 200 is configured to emit pulsed laser light, and the laser generator 200 has at least three different laser generation modes. The input device 400 is configured to input an instruction related to treatment part to the controller 100. The controller 100 is configured to receive the instruction and send out a corresponding control signal according to the pre-stored correspondence between treatment parts and the laser generation modes of the laser generator. The driving power supply 300 is configured to receive the control signal, and to drive, according to the control signal, the laser generator 200 to emit pulsed laser light in a corresponding laser generation mode. Since the laser generator 200 has at least three different laser generation modes, and the correspondence between treatment parts and the laser generation modes of the laser generator 200 is pre-stored in the controller 100, it is possible to use, according to specific conditions of the patient's treatment parts, any one of the at least three different laser generation modes for separate treatment or use any combination of them for combined treatment, so that different treatment plans can be applied to different treatment parts, and the therapeutic effect and application range of the laser therapy device can be effectively improved. Specifically, according to the specific conditions of the patient's treatment parts, the user can input an instruction related to treatment part and send the corresponding instruction to the controller 100 through the input device 400. The controller 100 is configured to send a corresponding control signal to the driving power supply 300 according to the pre-stored correspondence between treatment parts and the laser generation modes of the laser generator. The driving power supply 300 is configured to drive the laser generator 200 according to the received control signal. Driven by the driving power supply 300, the laser therapy device is configured to emit pulsed laser light in a corresponding laser generation mode.

For the description of the driving power supply 300 and the display screen, please refer to the description in Embodiment 1, which will not be repeated here.

As shown in Fig. 1, in some embodiments, the laser therapy device further includes a temperature sensor 500 connected to the controller 100. For the description of the temperature sensor here, please refer to the description in Embodiment 1, which will not be repeated here.

In some embodiments, the laser generator 200 includes three laser generation modes that are the harmonic pulse mode, fixed pulse mode and super pulse mode. Thus, the user, such as a doctor, can select a treatment part of the patient by using the above-mentioned display screen as the input device to input a corresponding instruction related to the treatment part, and the controller 100 is configured to, according to the received instruction, select these three modes to treat the patient's treatment part separately in different time periods to achieve a more effective therapeutic effect. In some other embodiments, the controller 100 is configured to, according to the received instruction, select one of the three laser generation modes or two of them to be used in different time periods to treat the treatment part of the patient, which is not limited here.

For specific descriptions related to the harmonic pulse mode, the fixed pulse mode and the super pulse mode, please refer to Figs. 2 to 7 in conjunction with the relevant content in the above-mentioned Embodiment 1, and will not be repeated here.

The operating principle and application scenarios of the laser therapy device according to this embodiment will be described below through specific examples.

Example 3

Since the correspondence between treatment parts and the laser generation modes of the laser generator 200 are pre-stored in the controller 100 of the laser therapy device according to this embodiment, different treatment plans can be provided for different treatment parts. Please refer to Fig. 11, which schematically shows an interface display about optional treatment parts according to an embodiment of the present invention. As shown in Fig. 11, five different treatment parts that are hands, back, legs, elbows and feet are included. The correspondence between the five different treatment parts (i.e., hands, back, legs, elbows and feet) and the laser generation modes of the laser generator 200 are stored in the controller 100. Thus, the user can select the corresponding treatment part according to the specific conditions of the patient, and send a corresponding instruction to the controller 100 through the display screen to provide a corresponding treatment plan. In order to facilitate the operation, there are indicating figures for different treatment parts on the display screen to prompt the user. The user can choose them according to the patient treatment part as needed. The specific display interface is shown in Fig. 11. It should be noted that, in this embodiment, hands, back, legs, elbows and feet are used as different treatment parts to illustrate the application scenarios of the laser therapy device according to this embodiment. However, those skilled in the art can be understood that the laser therapy device according to this embodiment can also be used to treat other parts of the human body, which is not limited in this embodiment.

Based on combinations of the above-mentioned three laser generation modes that are the harmonic pulse mode, fixed pulse mode and super pulse mode, laser treatment plans for different treatment parts are further proposed, as follows.

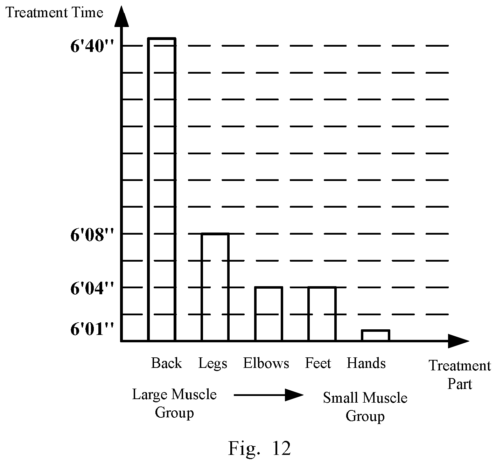

Please refer to Fig. 12, which schematically shows a comparison of laser treatment times for different treatment parts according to an embodiment of the present invention. As shown in Fig. 12, different treatment parts have different amounts of muscle, and the controller 100 is configured to control the total irradiation time of the laser light emitted from the laser generator 200 to decrease as the amounts of muscle in the treatment part decreases. Among the five treatment parts (i.e., the hands, back, legs, elbows and feet), the back is a large muscle group with the largest number of muscles, followed by the number of muscles in the legs, and elbows and feet have almost equal amount of muscles. The hands are small muscle groups with the least amount of muscles. Therefore, the treatment time (i.e., laser irradiation time) for the back is the longest, followed by the treatment time of the legs, and the treatment time for the elbows and feet are almost the same. The treatment time for the hands is the shortest.

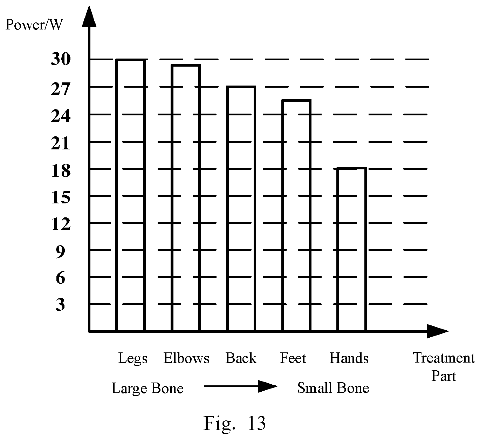

Please refer to Fig. 13, which schematically shows a comparison of laser output power for different treatment parts according to an embodiment of the present invention. As shown in Fig. 13, different treatment parts have different bone sizes, and the controller 100 is configured to control the laser output power of the laser generator 200 to decrease as the bone size of the treatment part decreases. Since the bone size of the legs, elbows, back, feet, and hands decreases sequentially, the output laser power of the laser light from the therapy device in the treatment for the legs is the largest; the output laser power of the laser light from the laser therapy device in the treatment for the elbows is the second largest; the laser power output of the laser light from the laser therapy device in the treatment for the back is lower than that for the elbows; the output laser power of the laser light from the laser therapy device in the treatment for the hands is the smallest; the output laser power of the laser light from the laser therapy device in the treatment for the feet is larger than that for the hands.

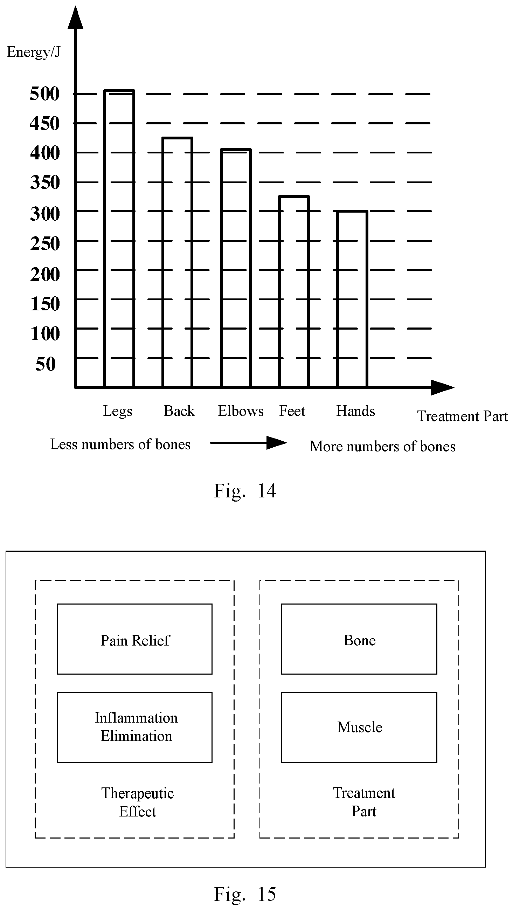

Please refer to Fig. 14, which schematically shows a comparison of laser output energies for different treatment parts according to an embodiment of the present invention. As shown in Fig. 14, different treatment parts have different numbers of bones, and the controller 100 is configured to control the laser output energy of the laser generator 200 to decrease as the number of bones in the treatment part increases. Since the number of bones in the legs, back, elbows, feet, and hands increases sequentially, the laser output energy of the laser generator in the treatment for the legs is the largest; the laser output energy of the laser generator in the treatment for the back is the second largest; the laser output energy of the laser generator in the treatment for the elbows is smaller than that for the back; the laser output energy of the laser generator in the treatment for the hands is the smallest; the laser output energy of the laser generator in the treatment for the feet is larger than that for the hands. For different parts such as legs, back, elbows, feet and hands, when treating any of them, three laser generation modes (i.e., the harmonic pulse mode, fixed pulse mode and super pulse mode) can be used in different stages/time periods. For example, the fixed pulse mode is used in a first stage, the super pulse mode is used in a second stage, and the harmonic pulse mode is used in a third stage.

For different treatment parts, the duration (i.e., the laser irradiation time) of the fixed pulse mode in the first stage is different, the durations of the super pulse mode in the second stage and the harmonic pulse mode in the third stage are the same, and the total duration for the three stages is 5-8 seconds, preferably 6-7 seconds, for example 6.02 seconds, 6.40 seconds.

Specifically, different treatment parts have different amounts of muscle, and the controller 100 is configured to control the laser irradiation time for the fixed pulse mode to decrease as the amounts of muscle decrease. Therefore, for the five treatment parts (i.e., hands, back, legs, elbows and feet), in the treatment for large muscle groups, such as the back, the duration of the fixed pulse mode (i.e., the laser irradiation time) is the longest, and in the treatment for small muscle groups, such as the hands, the duration of the fixed pulse mode is the shortest.

Different treatment parts have different bone sizes, and the controller 100 is configured to control the laser output power for the fixed pulse mode to decrease as the bone size of the treatment part decreases. Therefore, for the five treatment parts (i.e., hands, back, legs, elbows and feet), in the treatment for large bone parts, such as the legs, the laser output power for the fixed pulse mode is the largest, and in the treatment for small bone parts, such as the hand, the laser output power for the fixed pulse mode is the smallest, and the total laser output power for the three stages is 10-40W, preferably 15-30W, for example 15W, 30W.

Different treatment parts have different numbers of bones, and the controller 100 is configured to control the laser output energy for the fixed pulse mode to decrease as the number of bones in the treatment part increases. Therefore, for the five treatment parts (i.e., hands, back, legs, elbows and feet), in the treatment for the parts with a small number of bones, such as legs, the laser output energy for the fixed pulse mode is the largest, and in the treatment for the parts with a large number of bones, such as the hand, the laser output energy for the fixed pulse mode is the smallest, and the total laser output energy for the three stages is 200-600J, preferably 300-550J, for example 300J, 504J.

In addition to the treatment plan of using three different laser generation modes for treatment in this embodiment, those skilled in the art can also use any one of the above different laser generation modes for single treatment or use any combination of them for combined treatment according to the above ideas of the present invention, such as the tendency of the overall treatment time, power and energy for different treatment parts.

The laser therapy device according to this embodiment aims to effectively relieve muscle pain at the treatment part. Therefore, the inventors proposed the laser therapy device according to this embodiment in consideration of the following two aspects, the relevant biochemical indicators and the impact on endplate noise. The laser therapy device has provided remarkable therapeutic effect.

Tumor necrosis factor $\alpha$ (TNF-$\alpha$) is a cytokine released by immune cells, substance P is a signaling substance released by nociceptors, and cyclooxygenase (COX-2) is an enzyme that catalyzes the conversion of arachidonic acid, a pain-causing substance, into prostaglandins. These play an important role in the transmission of pain signals from peripheral nerves to the central nervous system. $\beta$G Endorphins ($\beta$GEP) is an endogenous opioid peptide produced by the pituitary gland, which can achieve endogenous analgesic effects by inhibiting the release of substance P from neurons. Combined with the results of clinical trials, it can be seen that the laser therapy device according to this embodiment can significantly reduce the content of substance P in the dorsal root ganglion after laser treatment, so that the muscles show lower TNF-$\alpha$ level and RNA expression of COX-2, and increased $\beta$GEP level of serum, muscle, dorsal root ganglion, so as to achieve the purpose of relieving muscle pain.

Spontaneous muscle electrical activity (SEA) is a basic feature of muscle pain, which is composed of endplate noise (EPN) and endplate spike potential. The occurrence rate of EPN in muscle pain point area is closely related to its sensitivity and excitability. When EPN occurs in large quantities, it will cause muscle pain. Combining with the results of clinical trials, it can be seen that after laser treatment using the laser therapy device according to this embodiment, the occurrence of EPN can be effectively inhibited, the occurrence rate of EPN can be greatly reduced, and the purpose of relieving muscle pain can be achieved.

In order to realize the above idea, this embodiment also provides a storage medium, which is applied to a laser therapy device whose laser generator has at least three different laser generation modes, and a computer program is stored in the storage medium. When the computer program is executed by a processor, it realizes:

sending out a corresponding laser generation control signal according to a received instruction and a pre-stored correspondence between treatment parts and the laser generation modes of the laser generator;
driving the laser generator to emit pulsed laser light in a corresponding laser generation mode according to the laser generation control signal.

The storage medium according to this embodiment can achieve a treatment by using a combination of at least three different laser generation modes of the laser generator according to the specific conditions of the treatment part of the patient.

In addition to the treatment plan of using three different laser generation modes for treatment in this embodiment, those skilled in the art can also use any one of the above different laser generation modes for single treatment or use any combination of them for combined treatment according to the above ideas of the present invention, such as the tendency of the overall treatment time, power and energy for different treatment parts. Therefore, different treatment plans can be applied to different treatment parts, and the therapeutic effect and application range of the laser therapy device can be effectively improved.

As to the specific form of the storage medium in this embodiment, please refer to the description in Embodiment 1, which will not be repeated here.

In some embodiments, the laser generator includes three laser generation modes that are the harmonic pulse mode, fixed pulse mode and super pulse mode.

In some embodiments, in the harmonic pulse mode, the laser generator emits n pulses at an equal pulse interval in one period, and the pulse widths of the n pulses are different, wherein n is a positive integer, and n≥2; in the fixed pulse mode, the laser generator emits n pulses with same pulse widths at different pulse intervals in one period, where n is a positive integer, and n≥3; in the super pulse mode, the laser generator emits n pulses with different pulse widths at different pulse intervals in one period, wherein n is a positive integer, and n A3.

In some embodiments, in the harmonic pulse mode and/or the super pulse mode, the pulse width $T_{N+1}$ of the $(N+1)^{th}$ pulse and the pulse width $T_N$ of the $N^{th}$ pulse satisfy the following equation:

$$T_{N+1} = \frac{1}{\theta} T_N \qquad (4)$$

wherein, $\theta$ is the average energy coefficient which is a positive integer equal to or greater than 2, and N is a positive integer.

In some embodiments, in the fixed pulse mode and/or in the super pulse mode, the pulse interval $t_k$ between the $(N+1)^{th}$ pulse and the $N^{th}$ pulse and the pulse interval $t_{k+1}$ between the $(N+2)^{th}$ pulse and the $(N+1)^{th}$ pulse satisfy the following equation:

$$t_{K+1} = \frac{1}{\xi} t_K \qquad (8)$$

wherein, $\xi$ is the average power coefficient which is a positive integer equal to or greater than 2, N is a positive integer, and K is a positive integer.

In some embodiments, when the computer program is executed by the processor, it realizes:
obtaining the temperature of the laser generator, and determining whether the temperature is higher than a preset threshold, and if so, performing overheating protection on the laser generator.

In some embodiments, different treatment parts have different amounts of muscle. As the amount of muscle in the treatment parts decreases, the total irradiation time of the laser light emitted from the laser generator decreases.

In some embodiments, different treatment parts have different bone sizes. As the bone size of the treatment part decreases, the total output power of the laser light emitted from the laser generator decreases.

In some embodiments, different treatment parts have different numbers of bones. As the number of bones in the treatment part increases, the total output energy of the laser light emitted from the laser generator decreases.

In some embodiments, different treatment parts have different amounts of muscle. As the amount of muscle in the treatment parts decreases, the laser irradiation time for the fixed pulse mode decreases.

In some embodiments, different treatment parts have different bone sizes. As the bone size of the treatment parts decreases, the laser output power for the fixed pulse mode decreases.

In some embodiments, different treatment parts have different numbers of bones. As the number of bones in the treatment parts increases, the laser output energy for the fixed pulse mode decreases.

In some embodiments, the treatment parts include hands, back, legs, elbows, and feet.

Further, three treatment stages are adopted for at least one of the treatment parts above, wherein the first stage adopts fixed pulse mode, the second stage adopts super pulse mode, and the third stage adopts harmonic pulse mode, and the three stages satisfy at least one of the followings:

the total laser irradiation time in the three stages for each part is 5-8 seconds;
the total laser output power in the three stages for each part is 10-40W;
the total laser output energy in the three stages for each part is 200-600J.

Furthermore, for different treatment parts, the laser irradiation time for the fixed pulse mode in the first stage is different, the laser irradiation times for the super pulse mode in the second stage, and the harmonic pulse mode in the third stage are same.

In conclusion, compared with the prior art, the laser therapy device and storage medium according to this embodiment have the following advantages. The laser therapy device according to this embodiment includes a controller, a laser generator and a driving power supply, wherein the laser generator has at least three different laser generation modes, and a correspondence between treatment parts and the laser generation modes of the laser generator is pre-stored in the controller. Therefore, it is possible to use, according to specific conditions of the patient, any one of the at least three different laser generation modes for separate treatment or any combination of them for combined treatment, so that different treatment plans can be applied to different treatment parts, and the therapeutic effect and application range of the laser therapy device can be effectively improved.

EMBODIMENT 3

The main object for this embodiment is to provide a laser therapy device and a storage medium, which can provide multiple laser generation modes, so that corresponding laser treatment plans can be applied to different therapeutic effects and/or treatment tissues.

As to the similarities between Embodiment 3 and Embodiments 1-2, please refer to the description in Embodiments 1-2, and details are not repeated here. That is to say, content not described in Embodiment 3 may refer to Embodiments 1-2 or other embodiments of the present invention. The differences between Embodiment 3 and Embodiments 1-2 are mainly described below.

In order to achieve the above idea, this embodiment provides a laser therapy device. Please refer to Fig. 1, which schematically shows a structural block diagram of the laser therapy device according to an embodiment of the present invention. For details, please refer to the description in Embodiment 1, which will not be repeated here.

The laser generator 200 is configured to emit pulsed laser light, and has at least three different laser generation modes. The input device 400 is configured to input an instruction related to therapeutic effect and/or treat tissue to the controller 100. The controller 100 is configured to receive the instruction and send out a corresponding control signal according to the pre-stored correspondence between therapeutic effects and/or the treated tissues and the laser generation modes of the laser generator. The driving power supply 300 is configured to receive the control signal, and drive, according to the control signal, the laser generator 200 to emit pulsed laser light in a corresponding laser generation mode. Since the laser generator 200 has at least three different laser generation modes, and the correspondence between therapeutic effects and/or treatment tissues and the laser generation modes of the laser generator 200 are pre-stored in the controller 100, it is possible to use, according to the therapeutic effect that the patient wants to achieve and/or the specific conditions of the treatment tissue, any one of the at least three different laser generation modes for separate treatment or use any combination of them for combined treatment, so that corresponding treatment plans can be provided for different therapeutic effects and/or treatment tissues, and the therapeutic effect and application range of the laser therapy device can be effectively improved. Specifically, according to the therapeutic effect that the patient wants to achieve and/or the specific conditions of the treatment tissue, the user can input an instruction related to the therapeutic effects and/or treatment tissues through the input device 400 and send the corresponding instruction to the controller 100. The controller 100 is configured to send out a corresponding control signal to the driving power supply 300 according to the correspondence between the pre-stored therapeutic effects and/or the treatment tissues and the laser generation modes of the laser generator. The driving power supply 300 is configured to drive the laser generator 200 according to the received control signal. The laser therapy device is configured to emit pulsed laser light in a corresponding laser generation mode under the drive of the driving power supply 300.

As to the description of the driving power supply 300 and the display screen, please refer to the description in Embodiment 1, which will not be repeated here.

As shown in Fig. 1, in some embodiments, the laser therapy device further includes a temperature sensor 500 connected to the controller 100. As to the description of the temperature sensor here, please refer to the description in Embodiment 1, which will not be repeated here.

In some embodiments, the laser generator 200 includes three laser generation modes that are the harmonic pulse mode, fixed pulse mode and super pulse mode. Thus, the user, such as a doctor, can select the therapeutic effect and/or treatment tissue that the patient wants by inputting a corresponding instruction related to the therapeutic effect and/or treatment tissue through the above-mentioned display screen serving as an input device, and the controller 100 is configured to control the three laser generation modes to be used separately in different time periods to treat the patient according to the received instruction, so as to achieve a more effective therapeutic effect. In some other embodiments, the controller 100 is configured to control one of the three laser generation modes to be used or two of them to be used separately in different time periods to treat the patient according to the received instruction, which is not limited in the present invention.

As to the specific descriptions related to the harmonic pulse mode, the fixed pulse mode and the super pulse mode, please refer to Figs. 2-7 in conjunction with the relevant content in the above-mentioned Embodiment 1, and will not be repeated here.

The operating principle and application scenarios of the laser therapy device according to this embodiment will be described below through specific examples.

Example 4

Since the correspondence between therapeutic effects and/or treatment tissues and the laser generation modes of the laser generator 200 are pre-stored in the controller 100 of the laser therapy device according to this embodiment, different treatment plans can be provided for different therapeutic effects and/or treatment tissues. Please refer to Fig. 15, which schematically shows a schematic diagram of the interface display about the therapeutic effects and the treatment tissues according to an embodiment of the present invention. As shown in Fig. 15, in this embodiment, the controller 100 stores the correspondence between the two therapeutic effects of pain relief and inflammation elimination and the two therapeutic tissues of muscle and bone and the laser generation modes of the laser generator 200. Thus, the user can select the corresponding therapeutic effect and treatment tissue according to the specific conditions of the patient, and send corresponding instructions to the controller 100 through the display screen to adopt the corresponding treatment plans. In order to facilitate the operation, indicating figures of different therapeutic effects and treatment tissues are displayed on the display screen to prompt the user. The user can choose according to the therapeutic effect and treatment organization that the patient wants. The display interface is shown in Fig. 15. It should be noted that, although the application scenarios of the laser therapy device according to this embodiment are described by taking pain relief and inflammation elimination as an example of the therapeutic effects and by taking muscle and bone as an example of the therapeutic tissues, a skilled person can understand that the laser therapy device according to this embodiment can also be used to achieve other therapeutic effects and/or treat other tissues, which is not limited in the present invention.

This embodiment is based on the above-mentioned three laser generation modes of harmonic pulse mode, fixed pulse mode and super pulse mode, and further according to the different combinations of the three different modes, a laser treatment plan based on different therapeutic effects and different treatment tissues is proposed, specifically as follows.

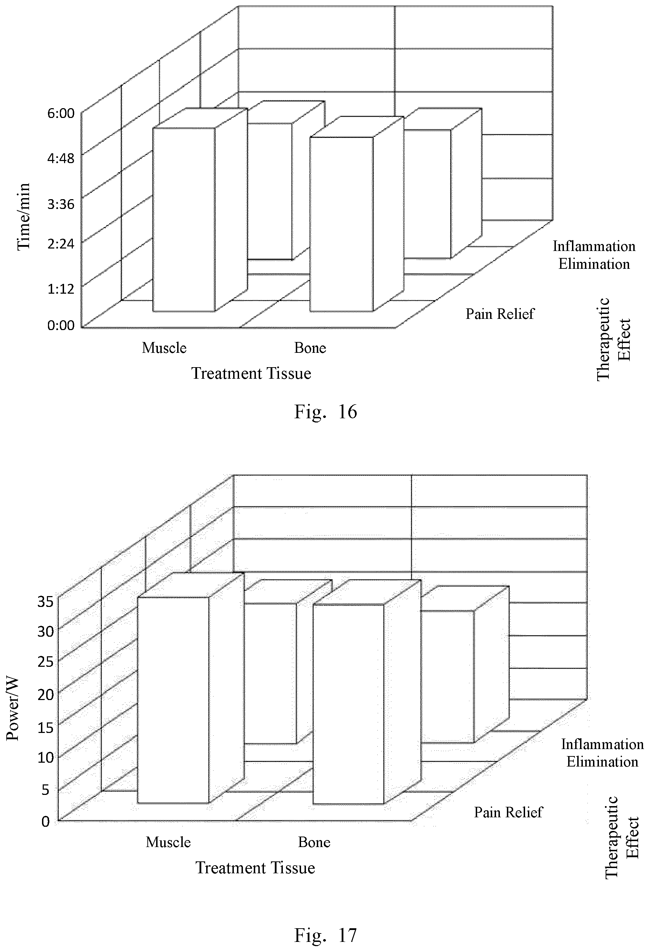

Please refer to Fig. 16, which schematically shows a comparison of laser treatment times for different therapeutic effects and different treatment tissues according to an embodiment of the present invention. As shown in Fig. 16, the controller 100 is configured to control the laser light emitted from the laser generator 200 so that the total irradiation time for pain relief is longer than the total irradiation time for inflammation elimination. Further, the controller 100 is configured to control the laser light emitted from the laser generator 200 so that the total irradiation time for treating muscle pain is longer than that the total irradiation time for treating bone pain, and the total irradiation time for treating muscle inflammation is longer than the total irradiation time for treating bone inflammation.

Please refer to Fig. 17, which schematically shows a comparison of laser output powers for different therapeutic effects and different treatment tissues according to an embodiment of the present invention. As shown in Fig. 17, the controller 100 is configured to control the laser light emitted from the laser generator 200 so that the total output power for pain relief is greater than the total output power for inflammation elimination. Further, the controller 100 is configured to control the laser light emitted from the laser generator 200 so that the total output power for treating muscle pain is greater than the total output power for treating bone pain, and the total output power for treating muscle inflammation is greater than the total output power for bone inflammation.

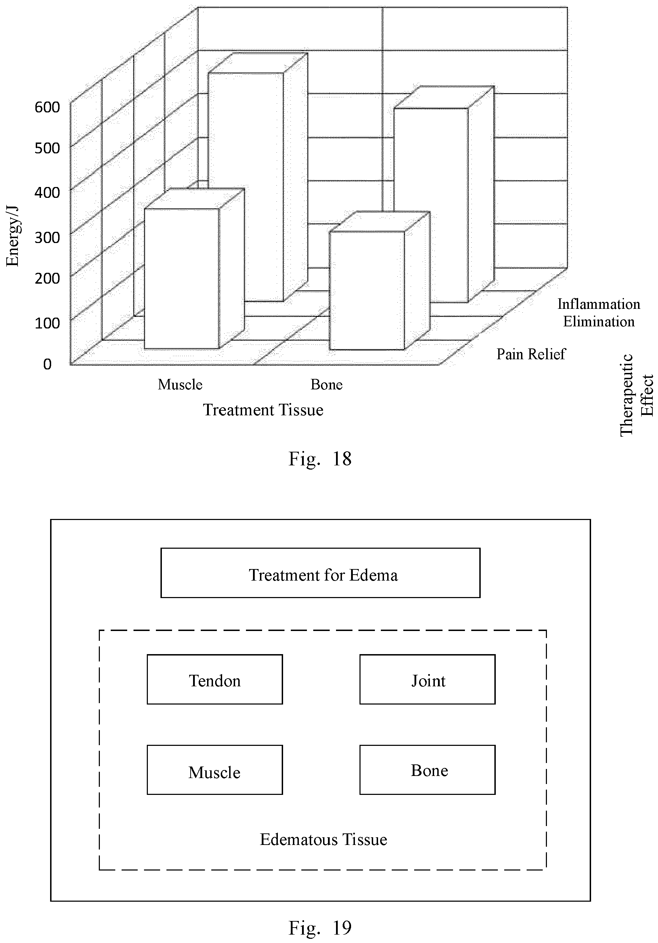

Please refer to Fig. 18, which schematically shows a comparison of laser output energies for different therapeutic effects and different treatment tissues according to an embodiment of the present invention. As shown in Fig. 18, the controller 100 is configured to control the laser light emitted from the laser generator 200 so that the total output energy for pain relief is smaller than the total output energy for inflammation elimination. Further, the controller 100 is configured to control the laser light emitted from the laser generator 200 so that the total output energy for treating muscle pain is larger than the total output energy for treating bone pain, and the total output energy for treating muscle inflammation is larger than the total output energy for treating bone inflammation.

Specifically, for the four different treatment objects of muscle pain, bone pain, muscle inflammation, and bone inflammation, when treating any of these objects, three laser generation modes (i.e., the harmonic pulse mode, fixed pulse mode and super pulse mode) can be used in different stages/time periods. For example, the fixed pulse mode is used in a first stage, the harmonic pulse mode is used in a second stage, and the super pulse mode is used in a third stage.

Specifically, when treating any one of the objects, the total laser irradiation time in the three stages ranges from 3 minutes to 6 minutes, such as 3.3 minutes, 4 minutes, 4.5 minutes, and 5 minutes. The laser irradiation time for the harmonic pulse mode in the second stage is the longest, the laser irradiation time for the super pulse mode in the third stage is the second longest, and the laser irradiation time for the fixed pulse mode in the first stage is the shortest.

When treating any one of the objects, the total laser output power in the three stages is 20W-40W, such as 21W, 26W, 31W, 32W. When treating muscle pain and bone pain, the laser output power for the super pulse mode in the third stage is the largest, the laser output power for the harmonic pulse mode in the second stage is the second, and the laser output power for the fixed pulse mode in the first stage is the smallest. When treating muscle inflammation and bone inflammation, the laser output power for the harmonic pulse mode in the second stage is the largest, the laser output power for the fixed pulse mode in the first stage is the second largest, and the laser output power for the super pulse mode in the third stage is the smallest.

When treating any one of the objects, the total laser output energy in the three stages is 200J-600J, such as 260J, 320J, 440J, 520J. When treating bone pain and muscle pain, the laser output energy for the fixed pulse mode in the first stage is the largest, the laser output energy for the super pulse mode in the third stage is the second, and the laser output energy for the harmonic pulse mode in the second stage is the smallest. When treating bone inflammation and muscle inflammation, the laser output energy for the super pulse mode in the third stage is the largest, the laser output energy for the fixed pulse mode in the first stage is the second largest, and the laser output energy for the harmonic pulse mode in the second stage is the smallest.

In order to realize the above idea, the present embodiment also provides a storage medium, which is applied to a laser therapy device whose laser generator has at least three different laser generation modes, and a computer program is stored in the storage medium. When the computer program is executed by a processor, it realizes:

sending out a corresponding laser generation control signal according to a received instruction and pre-stored correspondence between therapeutic effects/treatment tissues and the laser generation modes of the laser generator;

driving the laser generator to emit pulsed laser light in a corresponding laser generation mode according to the laser generation control signal.

The storage medium according to this embodiment can achieve a treatment by using a combination of at least three different laser generation modes of the laser generator according to the therapeutic effect that the patient wants to achieve and/or the specific conditions of the treated tissue.

In addition to the treatment plan of using at least three different laser generation modes for combined treatment in this embodiment, those skilled in the art can also use any one of the above different laser generation modes for single treatment or use any combination of them for combined treatment according to the above ideas of the present invention, such as the overall treatment time, power and energy for different therapeutic effects and/or treatment tissues. Therefore, different treatment plans can be applied for therapeutic effects and/or treatment tissues, and the therapeutic effect and application range of the laser therapy device can be effectively improved.

As to the specific form of the storage medium in this embodiment, please refer to the description in Embodiment 1, which will not be repeated here.

In some embodiments, the laser generator includes three laser generation modes that are the harmonic pulse mode, fixed pulse mode and super pulse mode.

In some embodiments, in the harmonic pulse mode, the laser generator emits n pulses at an equal pulse interval in one period, and the pulse widths of the n pulses are different, wherein n is a positive integer, and $n \geq 2$; in the fixed pulse mode, the laser generator emits n pulses with same pulse widths at different pulse intervals in one period, where n is a positive integer, and $n \geq 3$; in the super pulse mode, the laser generator emits n pulses with different pulse widths at different pulse intervals in one period, wherein n is a positive integer, and n A3.

In some embodiments, in the harmonic pulse mode and/or the super pulse mode, the pulse width $T_{N+1}$ of the $(N+1)^{th}$ pulse and the pulse width $T_N$ of the $N^{th}$ pulse satisfy the following equation:

$$T_{N+1} = \frac{1}{\theta} T_N \qquad (4)$$

wherein, $\theta$ is the average energy coefficient which is a positive integer equal to or greater than 2, and N is a positive integer.

In some embodiments, in the fixed pulse mode and/or in the super pulse mode, the pulse interval $t_k$ between the (N+1)th pulse and the Nth pulse and the pulse interval $t_{k+1}$ between the (N+2)th pulse and the (N+1)th pulse satisfy the following equation:

$$t_{K+1} = \frac{1}{\xi} t_K \qquad (8)$$

wherein, $\xi$ is the average power coefficient which is a positive integer equal to or greater than 2, N is a positive integer, and K is a positive integer.

In some embodiments, when the computer program is executed by the processor, it realizes: obtaining the temperature of the laser generator, and determining whether the temperature is higher than a preset threshold, and if so, performing overheating protection on the laser generator.

In some embodiments, the therapeutic effects include inflammation elimination and pain relief, and the treatment tissues include bones and muscles.

In some embodiments, for the laser light emitted from the laser generator, the total irradiation time for pain relief is longer than the total irradiation time for inflammation elimination.

In some embodiments, for the laser light emitted from the laser generator, the total irradiation time for treating muscle pain is longer than the total irradiation time for treating bone pain, and the total irradiation time for treating muscle inflammation is longer than the total irradiation time for treating bone inflammation.

In some embodiments, for the laser light emitted from the laser generator, the total output power for pain relief is greater than the total output power for inflammation elimination.

In some embodiments, for the laser light emitted from the laser generator, the total output power for treating muscle pain is greater than the total output power for treating bone pain, and the total output power for treating muscle inflammation is greater than the total output power for treating bone inflammation.

In some embodiments, for the laser light emitted from the laser generator, the total output energy for pain relief is smaller than the total output energy for inflammation elimination.

In some embodiments, for the laser light emitted from the laser generator, the total output energy for treating muscle pain is larger than the total output energy for treating bone pain, and the total output energy for treating muscle inflammation is larger than the total output energy for treating bone inflammation.

In some embodiments, when treating any of muscle pain, bone pain, muscle inflammation, and bone inflammation, three treatment stages are used, wherein the first stage adopts a fixed pulse mode, the second stage adopts a harmonic pulse mode, and the third stage adopts a super pulse mode, and the three stages satisfy at least one of the followings:

the total laser irradiation time in the three stages ranges from 3 minutes to 6 minutes, wherein, the laser irradiation time for the harmonic pulse mode in the second stage is the longest, and the laser irradiation time for the super pulse mode in the third stage is the second longest, the laser irradiation time for the fixed pulse mode in the first stage is the shortest;

the total laser output power in the three stages is 20W-40W, wherein, when treating muscle pain and bone pain, the laser output power for the super pulse mode in the third stage is the largest, the laser output power for the harmonic pulse mode in the second stage is the second largest, and the laser output power for the fixed pulse mode in the first stage is the smallest; when treating muscle inflammation and bone inflammation, the laser output power for the harmonic pulse mode in the second stage is the largest, the laser output power for the fixed pulse mode in the first stage is the second largest, and the laser output power for the super pulse mode in the third stage is the smallest;

the total laser output energy in the three stages is 200J-600J, wherein, when treating bone pain and muscle pain, the laser output energy for the fixed pulse mode in the first stage is the largest, the laser output energy for the super pulse mode in the third stage is the second largest, and the laser output energy for the harmonic pulse mode in the second stage is the smallest; when treating bone inflammation and muscle inflammation, the laser output energy for the super pulse mode in the third stage is the largest, the laser output energy for the fixed pulse mode in the first stage is the second largest, and the laser output energy for the harmonic pulse mode in the second stage is the smallest.

In conclusion, compared with the prior art, the laser therapy device and storage medium according to this embodiment have the following advantages. The laser therapy device according to this embodiment includes a controller, a laser generator and a driving power supply, wherein the laser generator has at least three different laser generation modes, and a correspondence between therapeutic effects and/or the treatment tissues and the laser generation modes of the laser generator is pre-stored in the controller. Therefore, it is possible to use, according to the therapeutic effect that the patient wants to achieve and/or the specific conditions of the treatment tissue, any one of the at least three different laser generation modes for separate treatment or any combination of them for combined treatment, so that different treatment plans can be applied for different therapeutic effects and/or treatment tissues, and the therapeutic effect and application range of the laser therapy device can be effectively improved.

EMBODIMENT 4

The main object for this embodiment is to provide a laser therapy device and a storage medium, which can provide at least two different laser generation modes, so that corresponding laser treatment schemes can be adopted for different edematous tissues.

As to the similarities between Embodiment 4 and Embodiments 1-3, please refer to the description in Embodiments 1-3, and details are not repeated here. That is to say, content not described in Embodiment 4 may refer to Embodiments 1-3 or other embodiments of the present invention. The differences between Embodiment 4 and Embodiments 1-3 are mainly described below.

In order to achieve the above idea, this embodiment provides a laser therapy device. Please refer to Fig. 1, which schematically shows a structural block diagram of the laser therapy device according to an embodiment of the present invention. For details, please refer to the description in Embodiment 1.

The laser generator 200 is configured to emit pulsed laser light, and has at least two different laser generation modes. The input device 400 is configured to input an instruction related to edematous tissue to the controller 100. The controller 100 is configured to receive the instruction and send out a corresponding control signal according to the pre-stored correspondence between instructions related to edema tissues and the laser generation modes of the laser generator 200. The driving power supply 300 is configured to receive the control signal, and drive, according to the control signal, the laser generator 200 to emit pulsed laser light in a corresponding laser generation mode. Since the laser generator 200 has at least two different laser generation modes, and the correspondence between instructions related to edema tissues and the laser generation modes of the laser generator 200 are pre-stored in the controller 100, it is possible to use, according to the specific conditions of the patient's edematous tissue, any one of the at least three different laser generation modes for separate treatment or use any combination of them for combined treatment, so that corresponding treatment plans can be provided for different edematous tissues, and the therapeutic effect and application range of the laser therapy device can be effectively improved. Specifically, according to the specific conditions of the patient's edematous tissue, the user can input an instruction related to edematous tissue through the input device 400 and send the corresponding instruction to the controller 100. The controller 100 is configured to send out a corresponding control signal to the driving power supply 300 according to the correspondence between the instructions related to edematous tissues and the laser generation modes of the laser generator. The driving power supply 300 is configured to drive the laser generator 200 according to the received control signal. The laser therapy device is configured to emit pulsed laser light in a corresponding laser generation mode under the drive of the driving power supply 300.

As to the description of the driving power supply 300 and the display screen, please refer to the description in Embodiment 1, which will not be repeated here.

As shown in Fig. 1, in some embodiments, the laser therapy device further includes a temperature sensor 500 connected to the controller 100. As to the description of the temperature sensor here, please refer to the description in Embodiment 1, which will not be repeated here.

In some embodiments, the laser generator 200 includes two laser generation modes that are the harmonic pulse mode and fixed pulse mode. Thus, the user, such as a doctor, can select the patient's edematous tissue by inputting a corresponding instruction related to the edematous tissue through the above-mentioned display screen serving as an input device, and the controller 100 is configured to control the two laser generation modes to be used separately in different time periods to treat the patient's edematous tissue according to the received instruction, so as to achieve a more effective therapeutic effect.

As to the specific descriptions related to the harmonic pulse mode and the fixed pulse mode, please refer to Figs. 2-5 in conjunction with the relevant content in the above-mentioned Embodiment 1, and will not be repeated here.

The operating principle and application scenarios of the laser therapy device according to this embodiment will be described below through specific examples.

Example 5

Since the correspondence between instructions related to edematous tissues and the laser generation modes of the laser generator 200 are pre-stored in the controller 100 of the laser therapy device according to this embodiment, corresponding treatment plans can be provided for various edematous tissues. Please refer to Fig. 19, which schematically shows a schematic view of an interface display about optional edematous tissues according to an embodiment of the present invention. As shown in Fig. 19, in this embodiment, four different edematous tissues that are tendons, muscles, joints and bones are included. The controller 100 stores the correspondence between instructions related to four different edematous tissues of tendons, muscles, joints and bones and the laser generation modes of the laser generator 200. Thus, the user can select the corresponding edematous tissue according to the specific conditions of the patient, and send corresponding instructions to the controller 100 through the display screen to adopt the corresponding treatment plans. In order to facilitate the operation, indicating figures of different edematous tissues are displayed on the display screen to prompt the user. The user can choose according to the patient's edematous tissue as needed. The specific display interface is shown in Fig. 19. It should be noted that although the application scenarios of the laser therapy device according to this embodiment are described by taking tendons, muscles, joints and bones as an example of the different edematous tissues, those skilled in the art can understand that the laser therapy device according to this embodiment can also be used to treat edema of other tissues of the human body, which is not limited in the present invention.

This embodiment is based on the above-mentioned two laser generation modes, i.e., the harmonic pulse mode and the fixed pulse mode, and further according to the combination of these two different modes, a laser treatment plan based on different edematous tissues is proposed, specifically as follows.

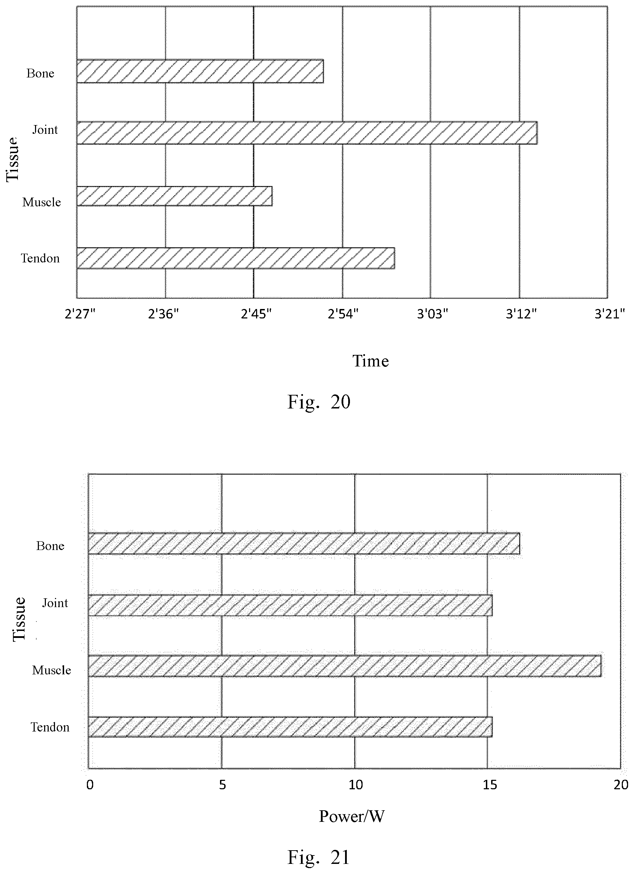

Please refer to Fig. 20, which schematically shows a comparison of laser irradiation times for different edematous tissues according to an embodiment of the present invention. As shown in Fig. 20, the controller 100 is configured to control the laser light emitted from the laser generator 200 so that the total irradiation time for treating joint edema is the longest, the total irradiation time for treating tendon edema is shorter than the total irradiation time for treating joint edema, the total irradiation time for treating bone edema is shorter than the total irradiation time for treating tendon edema, the total irradiation time for treating muscle edema is shorter than the total irradiation time for treating bone edema. Therefore, the total irradiation times of the laser light emitted from the laser generator 200 for treating joint edema, tendon edema, bone edema, and muscle edema decrease sequentially. It should be noted that, as those skilled in the art can understand, the total laser irradiation times for the treatments of joint edema, tendon edema, bone edema, and muscle edema shown in Fig. 20 is only a schematic illustration, and the present invention does not limit thereto.

Please refer to Fig. 21, which schematically shows a comparison of laser output powers for different edematous tissues according to an embodiment of the present invention. As shown in Fig. 21, the controller 100 is configured to control the laser light emitted from the laser generator 200 so that the total output power for treating muscle edema is the largest, the total output powers for treating bone edema and tendon edema decrease sequentially, and the total power output for treating joint edema is about the same as the total power output for treating tendon edema. It should be noted that, as those skilled in the art can understand, the total output power for treating joint edema, tendon edema, bone edema, and muscle edema shown in Fig. 21 is only a schematic illustration, and the present invention does not limit thereto.

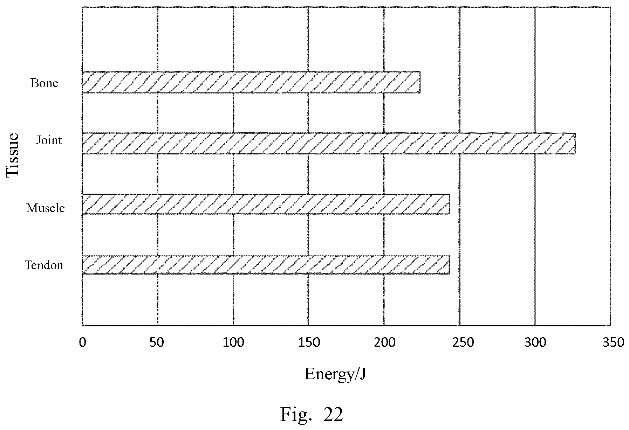

Please refer to Fig. 22, which schematically shows a comparison of laser output energies of different edematous tissues according to an embodiment of the present invention. As shown in Fig. 22, the controller 100 is configured to control the laser light emitted from the laser generator 200 so that the total output energy for treating joint edema is the largest, the total output energy for treating bone edema is the smallest, and the total output energy for treating muscle edema is about the same as the total output energy for treating tendon edema. It should be noted that, as can be understood by those skilled in the art, the total output energy for treating joint edema, tendon edema, bone edema, and muscle edema shown in Fig. 22 is only a schematic illustration, and the present invention does not limit thereto.

Specifically, the controller 100 is configured to control the laser generator 200 to emit laser light in a first stage and a second stage sequentially when treating any one of tendon edema, muscle edema, joint edema and bone edema, wherein the first stage adopts a fixed pulse mode, the second stage adopts a harmonic pulse mode, and the first stage and the second stage satisfy at least one of the followings:

the total laser irradiation time in the first stage and the second stage ranges from 2 minutes to 5 minutes, such as 2.3 minutes, 3 minutes, 4 minutes, 4.5 minutes, 5 minutes, wherein the laser irradiation time for the harmonic pulse mode in the second stage is longer than the laser irradiation time for the fixed pulse mode in the first stage;

the total laser output power in the first stage and the second stage ranges from 10W to 20W, such as 11W, 15W, 17W, 19W;

the total laser output energy in the first stage and the second stage ranges from 200J to 400J, such as 220J, 260J, 310J, 335J.

It can be seen that the laser therapy device according to this embodiment overcomes the problem that a traditional laser generator has a single control means. In this embodiment, under the effect of using the two laser generation modes, the therapeutic ability of the laser therapy device can be effectively enhanced, and the treatment capacity of the laser therapy device can be greatly improved. When the laser therapy device provided in this embodiment treats the edema of different tissues such as human tendons, muscles, joints and bones, two different laser generation modes are applied in this embodiment and corresponding treatment plans are provided, resulting in better therapeutic effect.

In order to realize the above idea, the present embodiment also provides a storage medium, which is applied to a laser therapy device whose laser generator has at least two different laser generation modes, and a computer program is stored in the storage medium. When the computer program is executed by a processor, it realizes:

sending out a corresponding laser generation control signal according to a received instruction and a pre-stored correspondence between instructions related to edematous tissues and the laser generation modes of the laser generator;

driving the laser generator to emit pulsed laser light in a corresponding laser generation mode according to the laser generation control signal.

The computer program in the storage medium according to this embodiment, when executed, can achieve a combined treatment by using any of at least two different laser generation modes of the laser generator according to the specific conditions of the patient's edematous tissue, so that corresponding treatment plans can be applied to different edematous tissues, and the therapeutic effect and application range of the laser therapy device can be effectively improved.

As to the specific form of the storage medium in this embodiment, please refer to the description in Embodiment 1, which will not be repeated here.

In some embodiments, the laser generator includes two laser generation modes that are the harmonic pulse mode and fixed pulse mode.

In some embodiments, in the harmonic pulse mode, the laser generator emits n pulses at an equal pulse interval in one period, and the pulse widths of the n pulses are different, wherein n is a positive integer, and n≥2; in the fixed pulse mode, the laser generator emits n pulses with same pulse widths at different pulse intervals in one period, where n is a positive integer, and n≥3.

In some embodiments, in the harmonic pulse mode, the pulse width $T_{N+1}$ of the (N+1)$^{th}$ pulse and the pulse width $T_N$ of the N$^{th}$ pulse satisfy the following equation:

$$T_{N+1} = \frac{1}{\theta} T_N \tag{4}$$

wherein, $\theta$ is the average energy coefficient which is a positive integer equal to or greater than 2, and N is a positive integer.

In some embodiments, in the fixed pulse mode, the pulse interval $t_k$ between the (N+1)$^{th}$ pulse and the N$^{th}$ pulse and the pulse interval $t_{k+1}$ between the (N+2)$^{th}$ pulse and the (N+1)$^{th}$ pulse satisfy the following equation:

$$t_{K+1} = \frac{1}{\xi} t_K \tag{8}$$

wherein, $\xi$ is the average power coefficient which is a positive integer equal to or greater than 2, N is a positive integer, and K is a positive integer.

In some embodiments, when the computer program is executed by the processor, it realizes:
obtaining the temperature of the laser generator, and determining whether the temperature is higher than a preset threshold, and if so, performing overheating protection on the laser generator.

In some embodiments, the edematous tissues include tendons, muscles, joints and bones.

In some embodiments, the total irradiation times of the laser light emitted from the laser generator for treating joint edema, tendon edema, bone edema, and muscle edema decrease sequentially.

In some embodiments, for the laser light emitted from the laser generator, the total output power for treating muscle edema is the largest, and the total output powers for treating bone edema and tendon edema decrease sequentially.

In some embodiments, for the laser light emitted from the laser generator, the total output energy for treating joint edema is the largest, and the total output energy for treating bone edema is the smallest.

In some embodiments, the laser generator is configured to emit laser light in a first stage and a second stage sequentially when treating any one of tendon edema, muscle edema, joint edema and bone edema, wherein the first stage adopts a fixed pulse mode, the second stage adopts a harmonic pulse mode, and the first stage and the second stage satisfy at least one of the followings:

the total laser irradiation time in the first stage and the second stage ranges from 2 minutes to 5 minutes, wherein the laser irradiation time for the harmonic pulse mode in the second stage is longer than the laser irradiation time for the fixed pulse mode in the first stage;
the total laser output power in the first stage and the second stage ranges from 10W to 20W;
the total laser output energy in the first stage and the second stage ranges from 200J to 400J.

In conclusion, compared with the prior art, the laser therapy device and storage medium according to this embodiment have the following advantages. The laser therapy device according to this embodiment includes a controller, an input device, a laser generator and a driving power supply, wherein the laser generator has at least two different laser generation modes, and a correspondence between instructions related to edematous tissues and the laser generation modes of the laser generator is pre-stored in the controller. Therefore, it is possible to use, according to specific conditions of the patient's edematous tissue, any of the at least two different laser generation modes for combined treatment, so that corresponding treatment plans can be applied to different edematous tissues, and the therapeutic effect and application range of the laser therapy device can be effectively improved.

The above description is only for the description of preferred embodiments of the present invention, and is not intended to limit the scope of the present invention. Any changes and modifications made by those skilled in the art according to the above disclosure are all within the protection scope of the appended claims. It is apparent that those skilled in the art can make various modifications and variations to the present invention without departing from the spirit and scope thereof. Accordingly, the invention is intended to embrace all such modifications and variations if they fall within the scope of the appended claims and equivalents thereof.

**Claims**

1. A laser therapy device, comprising a controller, a laser generator and a driving power supply, the driving power supply connected to the controller, and the laser generator connected to the driving power supply; wherein

the controller is configured to receive an instruction and send out a control signal;
the laser generator is configured to emit pulsed laser light, and the laser generator has at least two different laser generation modes;
the driving power supply is configured to receive the control signal, and drive the laser generator to emit pulsed laser light in a corresponding laser generation mode according to the control signal.

2. The laser therapy device according to claim 1, further comprising an input device connected to the controller, and the input device configured to send out an instruction related to laser generation mode, wherein the laser generator has three laser generation modes comprising a harmonic pulse mode, a fixed pulse mode and a super pulse mode.

3. The laser therapy device according to claim 2, wherein, in the harmonic pulse mode, the laser generator is configured to emit n pulses with different pulse widths at an equal pulse interval in one period, wherein n is a positive integer which is equal to or greater than 2.

4. The laser therapy device according to claim 2, wherein, in the fixed pulse mode, the laser generator is configured to emit n pulses with same pulse widths at different pulse intervals in one period, where n is a positive integer which is equal to or greater than 3.

5. The laser therapy device according to claim 2, wherein, in the super pulse mode, the laser generator is configured to emit n pulses with different pulse widths at different pulse intervals in one period, wherein n is a positive integer which is equal to or greater than 3.

6. The laser therapy device according to claim 3 or 5, wherein a pulse width $T_{N+1}$ of an $(N+1)^{th}$ pulse and a pulse width $T_N$ of an $N^{th}$ pulse satisfy:

$$T_{N+1} = \frac{1}{\theta} T_N$$

wherein, $\theta$ is an average energy coefficient which is a positive integer equal to or greater than 2, and N is a positive integer.

7. The laser therapy device according to claim 4 or 5, wherein a pulse interval $t_k$ between an $(N+1)^{th}$ pulse and an $N^{th}$ pulse and a pulse interval $t_{k+1}$ between an $(N+2)^{th}$ pulse and an $(N+1)^{th}$ pulse satisfy:

$$t_{K+1} = \frac{1}{\xi} t_K$$

wherein, $\xi$ is an average power coefficient which is a positive integer equal to or greater than 2, and N is a positive integer.

8. The laser therapy device according to claim 2, wherein the controller is configured to control the laser generator according to a pre-stored correspondence between patient skin tones and the laser generation modes of the laser generator.

9. The laser therapy device according to claim 8, wherein, as the patient skin tone gets darker, the controller is configured to control a total irradiation time, total output power and total output energy of the laser light emitted from the laser generator to decrease.

10. The laser therapy device according to claim 9, wherein, as the patient skin tone gets darker, the controller is configured to control the respective laser irradiation times for the harmonic pulse mode, the fixed pulse mode and the super pulse mode to decrease, wherein the laser irradiation time for the fixed pulse mode decreases the most.

11. The laser therapy device according to claim 9, wherein, as the patient skin tone gets darker, the controller is configured to control the respective laser output energies for the harmonic pulse mode, the fixed pulse mode and the super pulse mode to decrease, wherein the laser output energy for the fixed pulse mode decreases the most, the laser output energy for the harmonic pulse mode decreases the second most, and the laser output energy for the super pulse mode decreases the least.

12. The laser therapy device according to claim 2, wherein the controller is configured to control the laser generator according to a correspondence between patient age groups and the laser generation modes of the laser generator.

13. The laser therapy device according to claim 12, wherein, as the patient age increases, the controller is configured to control a total irradiation time, total output power and total output energy of the laser light emitted from the laser generator to increase.

14. The laser therapy device according to claim 12, wherein, as the patient age increases, the controller is configured to control the respective laser irradiation times for the harmonic pulse mode, the fixed pulse mode and the super pulse mode to increase, wherein the laser irradiation time for the fixed pulse mode increases the most.

15. The laser therapy device according to claim 12, wherein, as the patient age increases, the controller is configured to control the respective laser output energies for the harmonic pulse mode, the fixed pulse mode and the super pulse mode to increase, wherein the laser output energy for the fixed pulse mode increases the most, the laser output energy for the harmonic pulse mode increases the second most, and the laser output energy for the super pulse mode increases the least.

16. The laser therapy device according to claim 1, further comprising an input device connected to the controller; wherein

the laser generator is configured to emit pulsed laser light, and the laser generator has at least three different generation modes;
the input device is configured to input an instruction related to treatment part to the controller;
the controller is configured to receive the instruction and send out a corresponding control signal according to a pre-stored correspondence between treatment parts and the laser generation modes of the laser generator.

17. The laser therapy device according to claim 16, wherein the input device is a display screen configured for interface display and to send out an instruction for selecting treatment part, and the at least three different laser generation modes comprise a harmonic pulse mode, a fixed pulse mode and a super pulse mode.

18. The laser therapy device according to claim 16, wherein the input device is configured for a user to select different treatment parts with different amounts of muscle, wherein as the amount of muscle in the treatment part decreases, the controller is configured to control a total irradiation time of the laser light emitted from the laser generator to decrease.

19. The laser therapy device according to claim 16, wherein the input device is configured for a user to select different treatment parts with different bone sizes, wherein as the bone size of the treatment part decreases, the controller is configured to control a total output power of the laser light emitted from the laser generator to decrease.

20. The laser therapy device according to claim 16, wherein the input device is configured for a user to select different treatment parts with different numbers of bones, wherein as the number of bones in the treatment part increases, the controller is configured to control a total output energy of the laser light emitted from the laser generator to decrease.

21. The laser therapy device according to claim 17, wherein the input device is configured for a user to select different treatment parts with different amounts of muscle, wherein as the amount of muscle in the treatment part increases, the controller is configured to control a laser irradiation time for the fixed pulse mode to decrease.

22. The laser therapy device according to claim 17, wherein the input device is configured for a user to select different treatment parts with different bone sizes, wherein as the bone size of the treatment part decreases, the controller is configured to control a laser output power for the fixed pulse mode to decrease.

23. The laser therapy device according to claim 17, wherein the input device is configured for a user to select different treatment parts with different numbers of bones, wherein as the number of bones in the treatment part increases, the controller is configured to control a laser output energy for the fixed pulse mode to decrease.

24. The laser therapy device according to claim 17, wherein the treatment parts comprise hands, back, legs, elbows and feet.

25. The laser therapy device according to claim 16, wherein the controller is configured to adopt three treatment stages for at least one treatment part, wherein a first stage adopts a fixed pulse mode, a second stage adopts a super pulse mode, a third stage adopts a harmonic pulse mode, and the three stages satisfy at least one of the followings:

   a total laser irradiation time in the three stages for each treatment part is 5-8 seconds;
   a total laser output power in the three stages for each treatment part is 10-40W;
   a total laser output energy in the three stages for each treatment part is 200-600J.

26. The laser therapy device according to claim 25, wherein, the controller is configured to control the laser irradiation time for the fixed pulse mode in the first stage to be different for treating different treatment parts, and control the laser irradiation times for the super pulse mode in the second stage and the harmonic pulse mode in the third stage to be same for treating different treatment parts.

27. The laser therapy device according to claim 1, further comprising an input device connected to the controller; wherein

   the laser generator is configured to emit pulsed laser light, and the laser generator has at least three different laser generation modes;
   the input device is configured to input an instruction related to therapeutic effect and/or treatment tissue to the controller;
   the controller is configured to receive the instruction and send out a corresponding control signal according to a pre-stored correspondence between therapeutic effects and/or treatment tissues and the laser generation modes of the laser generator.

28. The laser therapy device according to claim 27, wherein the input device is a display screen configured for interface display and to send out an instruction for selecting therapeutic effect and/or therapeutic tissue, and the at least three different laser generation modes comprise a harmonic pulse mode, a fixed pulse mode and a super pulse mode.

29. The laser therapy device according to claim 17 or 28, wherein

in the harmonic pulse mode, the laser generator is configured to emit n pulses with different pulse widths at an equal pulse interval in one period, wherein n is a positive integer which is equal to or greater than 2;
in the fixed pulse mode, the laser generator is configured to emit n pulses with same pulse widths at different pulse intervals in one period, where n is a positive integer which is equal to or greater than 3;
in the super pulse mode, the laser generator is configured to emit n pulses with different pulse widths at different pulse intervals in one period, wherein n is a positive integer which is equal to or greater than 3.

30. The laser therapy device according to claim 29, wherein, in the harmonic pulse mode and/or the super pulse mode, a pulse width $T_{N+1}$ of an $(N+1)^{th}$ pulse and a pulse width $T_N$ of an $N^{th}$ pulse satisfy:

$$T_{N+1} = \frac{1}{\theta} T_N$$

wherein, $\theta$ is the average energy coefficient which is a positive integer equal to or greater than 2, and N is a positive integer.

31. The laser therapy device according to claim 29, wherein, in the fixed pulse mode and/or in the super pulse mode, a pulse interval $t_k$ between an $(N+1)^{th}$ pulse and an $N^{th}$ pulse and a pulse interval $t_{k+1}$ between an $(N+2)^{th}$ pulse and an $(N+1)^{th}$ pulse satisfy:

$$t_{K+1} = \frac{1}{\xi} t_K$$

wherein, $\xi$ is the average power coefficient which is a positive integer equal to or greater than 2, and N is a positive integer .

32. The laser therapy device according to claim 27, wherein the input device is configured for a user to select different therapeutic effects and treatment tissues, wherein the therapeutic effects comprise inflammation elimination and pain relief, and the treatment tissues comprise bones and muscles.

33. The laser therapy device according to claim 32, wherein the controller is configured to control the laser light emitted from the laser generator so that a total irradiation time of the laser light for pain relief is longer than a total irradiation time of the laser light for inflammation elimination.

34. The laser therapy device according to claim 33, wherein the controller is configured to control the laser light emitted from the laser generator so that a total irradiation time of the laser light for treating muscle pain is longer than a total irradiation time of the laser light for treating bone pain, and a total irradiation time of the laser light for treating muscle inflammation is longer than a total irradiation time of the laser light for treating bone inflammation.

35. The laser therapy device according to claim 32, wherein the controller is configured to control the laser light emitted from the laser generator so that a total output power of the laser light for pain relief is greater than a total output power of the laser light for inflammation elimination.

36. The laser therapy device according to claim 35, wherein the controller is configured to control the laser light emitted from the laser generator so that a total output power of the laser light for treating muscle pain is greater than a total output power of the laser light for treating bone pain, and a total output power of the laser light for treating muscle inflammation is greater than a total output power of the laser light for treating bone inflammation.

37. The laser therapy device according to claim 32, wherein the controller is configured to control the laser light emitted from the laser generator so that a total output energy of the laser light for pain relief is smaller than a total output energy of the laser light for inflammation elimination.

38. The laser therapy device according to claim 37, wherein the controller is configured to control the laser light emitted from the laser generator so that a total output energy of the laser light for treating muscle pain is larger than a total output energy of the laser light for treating bone pain, and a total output energy of the laser light for treating muscle inflammation is larger than a total output energy of the laser light for treating bone inflammation.

39. The laser therapy device according to claim 32, wherein the controller is configure to control the laser light emitted from the laser generator to treat any one of muscle pain, bone pain, muscle inflammation and bone inflammation in three stages, wherein a first stage adopts a fixed pulse mode, a second stage adopts a harmonic pulse mode, and a third stage adopts a super pulse mode, and the three stages satisfy at least one of the followings:

a total laser irradiation time in the three stages ranges from 3 minutes to 6 minutes, wherein, a laser irradiation time for the harmonic pulse mode in the second stage is the longest, and a laser irradiation time for the super pulse mode in the third stage is the second longest, a laser irradiation time for the fixed pulse mode in the first stage is the shortest;
a total laser output power in the three stages is 20W-40W, wherein, when treating muscle pain and bone pain, a laser output power for the super pulse mode in the third stage is the largest, a laser output power for the harmonic pulse mode in the second stage is the second largest, and a laser output power for the fixed pulse mode in the first stage is the smallest; when treating muscle inflammation and bone inflammation, a laser output power for the harmonic pulse mode in the second stage is the largest, a laser output power for the fixed pulse mode in the first stage is the second largest, and a laser output power for the super pulse mode in the third stage is the smallest;
a total laser output energy in the three stages is 200J-600J, wherein, when treating bone pain and muscle pain, a laser output energy for the fixed pulse mode in the first stage is the largest, a laser output energy for the super pulse mode in the third stage is the second largest, and a laser output energy for the harmonic pulse mode in the second stage is the smallest; when treating bone inflammation and muscle inflammation, a laser output energy for the super pulse mode in the third stage is the largest, a laser output energy for the fixed pulse mode in the first stage is the second largest, and a laser output energy for the harmonic pulse mode in the second stage is the smallest.

40. The laser therapy device according to claim 1, further comprising an input device connected to the controller; wherein

the laser generator is configured to emit pulsed laser light and has at least two different laser generation modes;
the input device is configured to input an instruction related to edematous tissue to the controller;
the controller is configured to receive the instruction and send out a corresponding control signal according to a pre-stored correspondence between instructions related to edematous tissues and the laser generation modes of the laser generator.

41. The laser therapy device according to claim 40, wherein the input device is a display screen configured for interface display and to send out an instruction for selecting edematous tissue, and the at least two different laser generation modes comprise a harmonic pulse mode and a fixed pulse mode.

42. The laser therapy device according to claim 41, wherein

in the harmonic pulse mode, the laser generator is configured to emit n pulses with different pulse widths at an equal pulse interval in one period, wherein n is a positive integer which is equal to or greater than 2;
in the fixed pulse mode, the laser generator is configured to emit n pulses with same pulse widths at different pulse intervals in one period, where n is a positive integer which is equal to or greater than 3.

43. The laser therapy device according to claim 42, wherein, in the harmonic pulse mode, a pulse width $T_{N+1}$ of an $(N+1)^{th}$ pulse and a pulse width $T_N$ of an $N^{th}$ pulse satisfy:

$$T_{N+1} = \frac{1}{\theta} T_N$$

wherein, $\theta$ is the average energy coefficient which is a positive integer equal to or greater than 2, and N is a positive integer.

44. The laser therapy device according to claim 42, in the fixed pulse mode, a pulse interval $t_k$ between an $(N+1)^{th}$ pulse and an $N^{th}$ pulse and a pulse interval $t_{k+1}$ between an $(N+2)^{th}$ pulse and an $(N+1)^{th}$ pulse satisfy:

$$t_{K+1} = \frac{1}{\xi} t_K$$

wherein, $\xi$ is the average power coefficient which is a positive integer equal to or greater than 2, and K is a positive integer.

45. The laser therapy device according to any one of claims 1, 16, 27 and 40, wherein the driving power supply is an adjustable constant current source, and the driving power supply is configured to receive the control signal and convert the control signal into a current signal to drive the laser generator to emit pulsed laser light in a corresponding mode.

46. The laser therapy device according to claim 40, wherein the input device is configured for a user to select different edematous tissues comprising tendons, muscles, joints and bones.

47. The laser therapy device according to claim 46, wherein the controller is configured to control total irradiation times of the laser light emitted from the laser generator for treating joint edema, tendon edema, bone edema, and muscle edema to decrease sequentially.

48. The laser therapy device according to claim 46, wherein the controller is configured to control the laser light emitted from the laser generator so that a total output power of the laser light for treating muscle edema is the largest, and total output powers of the laser light for treating bone edema and tendon edema decrease sequentially.

49. The laser therapy device according to claim 46, wherein the controller is configured to control the laser light emitted from the laser generator so that a total output energy of the laser light for treating joint edema is the largest, and a total output energy of the laser light for treating bone edema is the smallest.

50. The laser therapy device according to claim 46, wherein the controller is configured to control the laser light emitted from the laser generator to treat any one of tendon edema, muscle edema, joint edema and bone edema in a first stage and a second stage sequentially, wherein the first stage adopts a fixed pulse mode, the second stage adopts a harmonic pulse mode, and the first stage and the second stage satisfy at least one of the followings:

a total laser irradiation time in the first stage and the second stage ranges from 2 minutes to 5 minutes, wherein a laser irradiation time for the harmonic pulse mode in the second stage is longer than a laser irradiation time for the fixed pulse mode in the first stage;
a total laser output power in the first stage and the second stage ranges from 10W to 20W;
a total laser output energy in the first stage and the second stage ranges from 200J to 400J.

51. A storage medium, in which a computer program is stored, wherein
the computer program, when executed, realizes:

receiving an instruction and sending out a control signal;
driving a laser generator, according to the control signal, to emit pulsed laser light in at least two different laser generation modes.

52. The storage medium according to claim 51, wherein the laser generator comprises three different laser generation modes comprising a harmonic pulse mode, a fixed pulse mode and a super pulse mode; wherein

in the harmonic pulse mode, the laser generator is configured to emit n pulses with different pulse widths at an equal pulse interval in one period, wherein n is a positive integer which is equal to or greater than 2;
in the fixed pulse mode, the laser generator is configured to emit n pulses with same pulse widths at different pulse intervals in one period, where n is a positive integer which is equal to or greater than 3;
in the super pulse mode, the laser generator is configured to emit n pulses with different pulse widths at different pulse intervals in one period, wherein n is a positive integer which is equal to or greater than 3.

53. The storage medium according to claim 52, wherein a correspondence between patient skin tones and the laser generation modes of the laser generator is pre-stored in the storage medium.

54. The storage medium according to claim 52, wherein a correspondence between patient age groups and the laser generation modes of the laser generator is pre-stored in the storage medium.

55. The storage medium according to claim 51, wherein the laser generator has at least three different laser generation modes, and the computer program, when executed by a processor, realizes:

sending out a corresponding laser generation control signal according to a received instruction and a pre-stored correspondence between treatment parts and the laser generation modes of the laser generator;
driving the laser generator to emit pulsed laser light in a corresponding laser generation mode according to the laser generation control signal.

56. The storage medium according to claim 51, wherein the laser generator has at least three different laser generation modes, and the computer program, when executed by a processor, realizes:

sending out a corresponding laser generation control signal according to a received instruction and a pre-stored correspondence between therapeutic effects/treatment tissues and the laser generation modes of the laser generator;
driving the laser generator to emit pulsed laser light in a corresponding laser generation mode according to the laser generation control signal.

57. The storage medium according to claim 55 or 56, wherein the at least three different generation modes comprise a harmonic pulse mode, a fixed pulse mode and a super pulse mode; wherein

in the harmonic pulse mode, the laser generator is configured to emit n pulses with different pulse widths at an equal pulse interval in one period, wherein n is a positive integer which is equal to or greater than 2;
in the fixed pulse mode, the laser generator is configured to emit n pulses with same pulse widths at different pulse intervals in one period, where n is a positive integer which is equal to or greater than 3;
in the super pulse mode, the laser generator is configured to emit n pulses with different pulse widths at different pulse intervals in one period, wherein n is a positive integer which is equal to or greater than 3.

58. The storage medium according to claim 51, wherein the laser generator has at least two different laser generation modes, and the computer program, when executed by a processor, realizes:

sending out a corresponding laser generation control signal according to a received instruction and a pre-stored correspondence between instructions related to edematous tissues and the laser generation modes of the laser generator;
driving the laser generator to emit pulsed laser light in a corresponding laser generation mode according to the laser generation control signal.

59. The storage medium according to claim 58, wherein the at least two different laser generation modes comprise a harmonic pulse mode and a fixed pulse mode; wherein

in the harmonic pulse mode, the laser generator is configured to emit n pulses with different pulse widths at an equal pulse interval in one period, wherein n is a positive integer which is equal to or greater than 2;
in the fixed pulse mode, the laser generator is configured to emit n pulses with same pulse widths at different pulse intervals in one period, where n is a positive integer which is equal to or greater than 3.

Fig. 1

Fig. 2

Harmonic Pulse Mode

Power: auto

Unit: W

Energy: auto

Unit: J

Fig. 3

Fig. 4

Fixed Pulse Mode

| - Power + |

| Unit: W |

| - Energy + |

| Unit: J |

| - Frequency + |

| Unit: Hz |

Fig. 5

Fig. 6

Super Pulse Mode

| Power: auto | Energy: auto |
| Unit: W | Unit: J |

Fig. 7

The controller receives the instruction and sends out a control signal. — S1

The driving power supply receives the control signal, and drives the laser generator according to the control signal. — S2

Driven by the driving power supply, the laser generator emits pulsed laser light in a corresponding laser generation mode. — S3

Fig. 8

| <18 | 18-50 | 50-70 | >70 |

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

## INTERNATIONAL SEARCH REPORT

| International application No. |
| --- |
| **PCT/CN2021/120558** |

**A. CLASSIFICATION OF SUBJECT MATTER**

A61N 5/067(2006.01)n; A61B 18/20(2006.01)n

According to International Patent Classification (IPC) or to both national classification and IPC

**B. FIELDS SEARCHED**

Minimum documentation searched (classification system followed by classification symbols)

A61N5/-;A61B18/-

Documentation searched other than minimum documentation to the extent that such documents are included in the fields searched

Electronic data base consulted during the international search (name of data base and, where practicable, search terms used)

CNABS, CNTXT, CNKI, VEN, USTXT: 胡俊, 邸霈, 谢飞, 罗毅, 锐可医疗, 治疗, 理疗, 激光, 脉冲, 不同, 多种, 两种, 三种, 模式, 方式, 宽度, 脉宽, PW, 时间, 间隔, 相等, 相同, 不同, 不等, 肤色, 部位, 效果, 水肿, cure+, laser?, pulse?, impulse?, three, different, multi+, two, mode?, pulse length?, width?, time?, equal+, same?, color?, region?, effect?, dropsy?, edema?, oedema

**C. DOCUMENTS CONSIDERED TO BE RELEVANT**

| Category* | Citation of document, with indication, where appropriate, of the relevant passages | Relevant to claim No. |
| --- | --- | --- |
| PX | CN 112336452 A (RUIKE MEDICAL TECHNOLOGY (SHANGHAI) CO., LTD.) 09 February 2021 (2021-02-09) <br> claims 1-20 | 1-15, 45, 51-54 |
| PX | CN 112569477 A (RUIKE MEDICAL TECHNOLOGY (SHANGHAI) CO., LTD.) 30 March 2021 (2021-03-30) <br> claims 1-17 | 1-7, 16-52, 55-59 |
| PX | CN 112642063 A (RUIKE MEDICAL TECHNOLOGY (SHANGHAI) CO., LTD.) 13 April 2021 (2021-04-13) <br> claims 1-16 | 1-7, 16-52, 55-59 |
| PX | CN 112642065 A (RUIKE MEDICAL TECHNOLOGY (SHANGHAI) CO., LTD.) 13 April 2021 (2021-04-13) <br> claims 1-13 | 1, 40-51, 58-59 |
| X | CN 105456024 A (INSTITUTE OF BIOMEDICAL ENGIEERING, CHINESE ACADEMY OF MEDICAL SCIENCES) 06 April 2016 (2016-04-06) <br> description, paragraphs [0010]-[0019], and figures 1-6 | 1, 45, 51 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 November 2021** | **07 December 2021** |

| Name and mailing address of the ISA/CN | Authorized officer |
| --- | --- |
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/120558** |

**C.    DOCUMENTS CONSIDERED TO BE RELEVANT**

| Category* | Citation of document, with indication, where appropriate, of the relevant passages | Relevant to claim No. |
|---|---|---|
| Y | CN 105456024 A (INSTITUTE OF BIOMEDICAL ENGIEERING, CHINESE ACADEMY OF MEDICAL SCIENCES) 06 April 2016 (2016-04-06) description, paragraphs [0010]-[0019], and figures 1-6 | 2-44, 46-50, 52-59 |
| Y | US 2014121631 A1 (SEMINEX CORP.) 01 May 2014 (2014-05-01) description, paragraphs [0084]-[0088], and figures 10-12 | 2-44, 46-50, 52-59 |
| X | CN 205569065 U (SHANGHAI MUORANGE MEDICAL DEVICES CO., LTD.) 14 September 2016 (2016-09-14) description paragraphs [0016]-[0026], figures | 1, 45, 51 |
| X | EP 1106209 A1 (RGM SPA) 13 June 2001 (2001-06-13) description, paragraphs [0001]-[0013], and figure 1 | 1, 45, 51 |
| X | CN 109818236 A (SHANGHAI JINGUANGROU BIOTECHNOLOGY CO., LTD.) 28 May 2019 (2019-05-28) description, paragraphs [0008]-[0059], and figure 3 | 1, 45, 51 |
| X | CN 208541431 U (XIE, Jianhao) 26 February 2019 (2019-02-26) description, paragraphs [0001]-[0059], and figure 1 | 1, 45, 51 |
| A | CN 107537097 A (XU, Chunguang) 05 January 2018 (2018-01-05) entire document | 1-59 |
| A | CN 201832290 U (B & W TEK OPTO-ELECTRONICS EQUIPMENT CO., LTD.) 18 May 2011 (2011-05-18) entire document | 1-59 |
| A | US 6443978 B1 (UNIVERSITY OF ARKANSAS) 03 September 2002 (2002-09-03) entire document | 1-59 |

Form PCT/ISA/210 (second sheet) (January 2015)

<table>
<tr><td colspan="2"><strong>INTERNATIONAL SEARCH REPORT</strong><br>Information on patent family members</td><td colspan="2">International application No.<br><strong>PCT/CN2021/120558</strong></td></tr>
</table>

| Patent document cited in search report | | | Publication date (day/month/year) | Patent family member(s) | | | Publication date (day/month/year) |
|---|---|---|---|---|---|---|---|
| CN | 112336452 | A | 09 February 2021 | None | | | |
| CN | 112569477 | A | 30 March 2021 | None | | | |
| CN | 112642063 | A | 13 April 2021 | None | | | |
| CN | 112642065 | A | 13 April 2021 | None | | | |
| CN | 105456024 | A | 06 April 2016 | None | | | |
| US | 2014121631 | A1 | 01 May 2014 | EP | 2914193 | A1 | 09 September 2015 |
| | | | | EP | 2914193 | B1 | 28 July 2021 |
| | | | | US | 2016250498 | A1 | 01 September 2016 |
| | | | | US | 9333371 | B2 | 10 May 2016 |
| | | | | WO | 2014070699 | A1 | 08 May 2014 |
| CN | 205569065 | U | 14 September 2016 | None | | | |
| EP | 1106209 | A1 | 13 June 2001 | IT | GE990141 | A1 | 11 June 2001 |
| | | | | IT | 1309464 | B1 | 23 January 2002 |
| CN | 109818236 | A | 28 May 2019 | None | | | |
| CN | 208541431 | U | 26 February 2019 | None | | | |
| CN | 107537097 | A | 05 January 2018 | None | | | |
| CN | 201832290 | U | 18 May 2011 | None | | | |
| US | 6443978 | B1 | 03 September 2002 | RU | 2145247 | C1 | 10 February 2000 |
| | | | | WO | 9952597 | A1 | 21 October 1999 |
| | | | | CN | 1296420 | A | 23 May 2001 |
| | | | | RU | 2195981 | C2 | 10 January 2003 |
| | | | | EP | 1074275 | A1 | 07 February 2001 |
| | | | | EP | 1074275 | A4 | 12 February 2003 |
| | | | | JP | 2002511323 | A | 16 April 2002 |
| | | | | CA | 2325928 | A1 | 21 October 1999 |
| | | | | AU | 3542599 | A | 01 November 1999 |
| | | | | AU | 750260 | B2 | 11 July 2002 |

Form PCT/ISA/210 (patent family annex) (January 2015)